# EUROPEAN PATENT APPLICATION

(11) **EP 4 108 252 A1**
(43) Date of publication of application: **28.12.2022**
(21) Application number: 21305865.4
(22) Date of filing: 23.06.2021
(51) Int. Cl.: A61K 38/26, A61K 9/00, A61P 19/02

(54) **PHARMACEUTICAL COMPOSITIONS COMPRISING GLP-1R AGONISTS**

(71) Applicant: 4Moving Biotech, 59000 Lille (FR)
(72) Inventor: Rattenbach, Revital, 75004 Paris (FR); Berenbaum, Francis, 91190 Gif sur Yvette (FR); Bismuth, Keren, 75011 Paris (FR); Martin, Céline, 59175 Templemars (FR); Meurot, Coralie, 59190 Hondeghem (FR)
(74) Representative: Icosa

(57) **Abstract**

The present invention relates to a pharmaceutical composition comprising a GLP-1R agonist such as liraglutide, a buffer selected from a tromethamine buffer and a phosphate buffer, and an isotonic agent selected from glucose, a polyethylene glycol and glycerol. The present invention also relates to said pharmaceutical composition for use in a method for treating joint diseases.

## Description

### FIELD OF INVENTION

The present invention relates to pharmaceutical compositions comprising GLP-1R agonists such as liraglutide. The present invention also relates to pharmaceutical compositions comprising GLP-1R agonists such as liraglutide for use in the treatment of joint diseases, the pharmaceutical compositions being administered, for example, via intraarticular injection.

### BACKGROUND OF INVENTION

Glucagon Like Peptide-1 (GLP-1) is a peptide hormone that binds to GLP-1 receptors expressed on the pancreatic beta cells, thus increasing the glucose transporter 2 expression and the secretion of insulin in response to increased blood glucose concentration. Moreover, GLP-1 reduces the secretion of some proinflammatory cytokines. GLP-1R agonists are commonly used as a treatment for type 2 diabetes.

Among the chronic joint diseases, osteoarthritis (OA) is the most prevalent disease, affecting nearly 50% of people over the age of 65 and occurring in younger people in case of anatomical abnormality, following a joint injury or in case of obesity. Worldwide, about 250 million people suffer from OA; this disease has major economic and social impacts.

Recently, it has been found that GLP-1R agonists such as liraglutide target relevant mechanism associated with inflammatory and regenerative processes relevant to OA. WO2020104833 thus relates to pharmaceutical compositions comprising GLP-1R agonists such as liraglutide, for use in the treatment of joint diseases, for example OA. The pharmaceutical compositions according to WO2020104833 are in particular in the form of gels that comprise liraglutide and albumin.

However, there is a need to find new formulations comprising GLP-1R agonists such as liraglutide, that would be at least as effective as the formulations already on the market, while being able to induce a long-term effect when administered via intraarticular injection, in particular an effect that would last at least three weeks. Indeed, limiting the frequency of intra-articular injections would both simplify the treatment for the patient and improve his or her comfort, and limit the number of medical procedures requiring the intervention of a caregiver.

The inventors have surprisingly found that the formulations of pharmaceutical compositions comprising GLP-1R agonists such as liraglutide according to the present invention are able to induce a long-term effect when administered via intraarticular injection, in particular an effect that lasts at least three weeks.

### SUMMARY

The present invention relates to a pharmaceutical composition for use in a method for treating joint diseases, in particular osteoarthritis and/or joint pain, more particularly inflammatory joint pain, wherein said pharmaceutical composition is to be administered via intraarticular injection, in particular via intraarticular injection into the joint cavity, and wherein said pharmaceutical composition comprises:
- a GLP-1R agonist,
- a buffer selected from the group consisting of a tromethamine buffer and a phosphate buffer, and
- an isotonic agent selected from the group consisting of glucose, a polyethylene glycol, propylene glycol and glycerol.

In one embodiment, the GLP-1R agonist is liraglutide, the buffer is a phosphate buffer, preferably a phosphate buffer comprising disodium phosphate dihydrate as buffering agent, and the isotonic agent is propylene glycol.

In another embodiment, said pharmaceutical composition comprises:
- a GLP-1R agonist, preferably the GLP-1R agonist is liraglutide,
- a buffer selected from the group consisting of a tromethamine buffer and a phosphate buffer, and
- an isotonic agent selected from the group consisting of glucose, a polyethylene glycol and glycerol.

Preferably, the GLP-1R agonist is liraglutide and said pharmaceutical composition is to be administered at a dose from 0.0245 mg to 6.3 mg of liraglutide, preferably at a dose from 0.7 mg to 6.3 mg, of liraglutide.

Advantageously, said dose of said pharmaceutical composition is to be administered in one or at least two intraarticular injections.

Preferably, doses of said pharmaceutical composition are to be administered every month.

Advantageously, the total dose of GLP-1R agonist that is administered in one year is from 0.18 mg to 72 mg, preferably from 0.7 mg to 8.4 mg.

The present invention also relates to a pharmaceutical composition comprising:
- a GLP-1R agonist,
- a buffer selected from the group consisting of a tromethamine buffer and a phosphate buffer, and
- an isotonic agent selected from the group consisting of glucose, a polyethylene glycol and glycerol.

Preferably, the GLP-1R agonist is liraglutide.

Advantageously, the pharmaceutical composition comprises from 2 to 20 mg/mL, preferably from 4 to 8 mg/mL, more preferably about 6 mg/mL of GLP-1R agonist.

In one embodiment, the buffer is a tromethamine buffer comprising tromethamine as buffering agent, preferably the pharmaceutical composition comprises from 0.1 to 10 mg/mL, more preferably from 0.5 to 1 mg/mL, even more preferably about 0.97 mg/mL of tromethamine.

In another embodiment, the buffer is a phosphate buffer comprising disodium phosphate as buffering agent, preferably the pharmaceutical composition comprises from 0.1 to 10 mg/mL, more preferably from 0.75 to 1,5 mg/mL, even more preferably about 1,14 mg/mL of disodium phosphate.

In one embodiment, the isotonic agent is glucose, preferably said pharmaceutical composition comprises from 10 to 50 mg/mL, more preferably from 20 to 40 mg/mL, even more preferably about 30 mg/mL of glucose.

In another embodiment, the isotonic agent is a polyethylene glycol having a molecular weight less than 800 g.mol⁻¹, preferably from 100 g.mol⁻¹ to 600 g.mol⁻¹, preferably the isotonic agent is PEG400, and
wherein said pharmaceutical composition comprises from 20 to 100 mg/mL, preferably from 40 to 80 mg/mL, more preferably about 60 mg/mL of polyethylene glycol.

In another embodiment, said pharmaceutical composition comprises from 5 to 50 mg/mL, preferably from 10 to 25 mg/mL, more preferably about 17 mg/mL or about 18 mg/mL of glycerol.

### DEFINITIONS

In the present invention, the following terms have the following meanings:
**"About",** before a figure or number, refers to plus or minus 10% of the face value of that figure or number. In one embodiment, "about", before a figure or number, refers to plus or minus 5% of the face value of that figure or number.
**"Active agent"** refers to an agent that has a therapeutic effect. The agent may be a chemical or a biological substance. The therapeutic effect may be the prevention, delay, reduction in severity and/or frequency or suppression of at least one symptom associated with a pathological condition, or the prevention, slowing down or suppression of the underlying cause of a pathological condition, or the improvement or repair of a damage.
**"Acute disease"** refers to a non-chronic disease.
**"Administration in combination"** refers to sequential, simultaneous or separate administration. When the administration in combination is simultaneous, the substances administered simultaneously may be present in the same pharmaceutical composition.
**"Buffer"** relates to a mixture of a weak acid and its conjugate base, or a weak base and its conjugate acid, whose pH is maintained constant when a small amount of strong acid or base is added to it. In an embodiment, the buffer is a phosphate buffer. In an embodiment, the buffer is a tromethamine buffer. A **"phosphate buffer"** is a buffer which comprises phosphate or a derivative thereof as buffering agent. A **"tromethamine buffer"** is a buffer which comprises tromethamine or a derivative thereof as buffering agent.
**"Buffering agent"** refers to the specific chemical that is present in both an acidic and a basic forms in a buffer, allowing the pH of a pharmaceutical composition to be maintained constant. In an embodiment, the buffering agent is phosphate or a derivative thereof. In an embodiment, the buffering agent is tromethamine or a derivative thereof.
**"Cartilage"** or **"cartilage matrix"** or **"articular cartilage"** refers to elastic, translucent connective tissue in mammals, including human. Cartilage comprises chondrocytes, type II collagen, small amounts of other collagen types, other noncollagenous proteins, proteoglycans and water. Although most cartilage becomes bone upon maturation, some cartilage remains in its original form in some locations, such as the nose, ears, knees. The cartilage has no blood or nerve supply.
**"Chronic disease"** refers to a long-term, progressive illness, often associated with disability and the threat of serious complications. Chronic diseases evolve more or less rapidly for at least several months, in particular at least 3 months.
**"Complex of GLP-1R agonist"** refers to a polyatomic structure consisting of one or more independent entities (ions or molecules), in interaction, said structure comprising a GLP-1R agonist.
**"Comprising"** or **"comprise"** is to be construed in an open, inclusive sense, but not limited to. In an embodiment, "comprising" means "consisting essentially of". In an embodiment, "comprising" means "consisting of', which is to be construed as limited to.
**"Dose"** refers to the cumulative amount of GLP-1R agonists administered in 2 weeks to 1 month. In one embodiment, one **"dose"** refers to the cumulative amount of GLP-1R agonists administered in 2 weeks. In another embodiment, one **"dose"** refers to the cumulative amount of GLP-1R agonists administered in 3 weeks. In another embodiment, one **"dose"** refers to the cumulative amount of GLP-1R agonists administered in 1 month.
**"Effective amount"** of an active agent refers to a nontoxic but sufficient amount of said active agent to provide the desired therapeutic effect.
**"Excipients"** refers to any inactive ingredient which is required for the formulation of an active agent in a suitable dosage form. In one embodiment, "Excipients" refers to any and all solvents, diluents carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, or any combination thereof. The person skilled in the art knows how to choose suitable excipients to obtain a formulation suitable for intra-articular injection, particularly in terms of viscosity, solvent, etc.
**"From X to Y"** refers to the range of values between X and Y, the limits X and Y being included in said range.
**"GLP-1"** or **"glucagon-like peptide 1"** refers to a 30- or 31-amino acid long peptide hormone deriving from the post-translational processing of the proglucagon peptide into **"GLP-1 (1-37)",** which is further *N*-terminally truncated by tissue-specific post-translational processing in the intestinal L cells resulting in the two truncated and equipotent biologically active forms, **"GLP-1 (7-36) amide"** and **"GLP-1 (7-37)".** In humans, GLP-1 (1-37) has an amino acid sequence as set forth in SEQ ID NO: 1; GLP-1 (7-36) amide has an amino acid sequence as set forth in SEQ ID NO: 2; and GLP-1 (7-37) has an amino acid sequence as set forth in SEQ ID NO: 3.
"**GLP-1R agonists"** or **"GLP-1 receptor agonists"** refers to agonists of the GLP-1 receptor (GLP1R). GLP-1R agonists may be GLP-1 analogues. GLP-1R agonists may be selected from the group consisting of liraglutide, exenatide, lixisenatide, albiglutide, beinaglutide, dulaglutide, semaglutide, pegapamodutide and taspoglutide. According to the present invention, the term "GLP-1G agonists" include GLP-1R agonists enantiomers, GLP-1R agonists esters, GLP-1R agonists racemates, salts of GLP-1R agonists, solvates of GLP-1R agonists, hydrates of GLP-1R agonists, polymorphs of GLP-1R agonists and complexes of GLP-1R agonists.
"**GLP-1R agonist enantiomer"** refers to a molecule that has the same molecular formula and sequence of bonded atoms as a GLP-1R agonist, but differs from said GLP-1R agonist in the three-dimensional orientation of its atoms in space, the GLP-1R agonist and its enantiomer being mirror images of each other and non-superposable.
"**GLP-1R agonist ester"** refers to a GLP-1R agonist bonded to another chemical molecule via an ester group.
"**GLP-1R agonist racemate"** refers to a mixture in equal proportions of the levorotatory and dextrorotatory enantiomers of the GLP-1R agonist.
**"Hydrate of a compound"** refers to a molecular complex comprising the compound and one or more pharmaceutically acceptable solvent molecules, wherein the solvent is water.
**"Intraarticular injection"** refers to an injection directly into the closed cavity of a joint in the human body.
**"Isotonic agent"** refers to an agent added to a pharmaceutical composition to ensure isotonicity between the pharmaceutical composition and the biological medium in which the pharmaceutical composition is administered.
**"Joint"** and **"articulation"** are used interchangeably.
**"Joint disease"** refers to any disease affecting at least one joint in a human body. Examples of joint diseases include, but are not limited to, inflammatory arthritis (in particular osteoarthritis, rheumatoid arthritis, psoriatic arthritis, juvenile arthritis, ankylosing spondylitis, lupus and related connective tissue diseases, synovitis crystal arthropathies (gout, chondrocalcinosis, oochronsis, hydroxyapatitis), abarticular pathologies (tendinitis, capsulitis, enthesitis), septic arthritis, subchondral bone pathologies (osteonecrosis, insufficiency fracture, bone marrow lesions), genetic arthropathies, inflammatory joint pain or any other joint pains. Joint diseases can be characterized by one or several symptoms including, without limitation, limitation of motion, joint pain, joint inflammation, joint tenderness, joint stiffness, and joint swelling.
**"Liraglutide"** refers to a 32-amino acid peptide of 3.7 kDa. It is a synthetic acylated analog of human GLP-1 (7-37), in which the lysine residue at position 28 (SEQ ID NO: 3 numbering) is replaced by an arginine residue, and a C16 fatty acid (palmitic acid) is bound to the ε-amino group of the lysine residue at position 20 (SEQ ID NO: 3 numbering) through a γ-glutamyl linker. The chemical structure of liraglutide is: 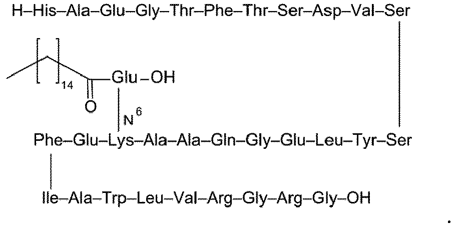
**"Osteoarthritis"** or **"OA"** is a joint disease. Osteoarthritis is a disorder that can affect any moveable joint of the body, for example knees, hips, and/or hands. It can show itself as a breakdown of tissues and abnormal changes to cell structures of joints, which can be initiated by injury. As the joint tries to repair, it can lead to other problems. Osteoarthritis first shows itself as a change to the biological processes within a joint, followed by abnormal changes to the joint, such as the breakdown of cartilage, bone reshaping, bony lumps, joint inflammation, and loss of joint function. This can result in pain, stiffness and loss of movement. There are certain factors which make some people more vulnerable to developing osteoarthritis, such as genetic factors, other joint disorders (such as rheumatoid arthritis), injury to the joint from accidents or surgery, being overweight or doing heavy physical activity in some sports or a person's job.
**"Pharmaceutical composition"** refers to the combination of at least one active agent and at least one pharmaceutically acceptable excipient.
**"Pharmaceutically acceptable"** refers to generally safe, non-toxic and neither biologically nor physiologically nor otherwise undesirable for animals, in particular for humans.
**"Polymorph of GLP-1R agonist"** refers to another crystal structure of said GLP-1R agonist.
**"Preservative"** refers to any substance or chemical that is added to a composition to prevent its decomposition by microbial growth or by undesirable chemical changes.
**"Salts of GLP-1R agonist"** refers to acid or base addition salts of GLP-1R agonist. The acid addition salts are formed with pharmaceutically acceptable organic or inorganic acids; the base addition salts are formed when an acid proton present in the GLP-1R agonist is either replaced by a metal ion or coordinated with a pharmaceutically acceptable organic or inorganic base.
**"Solvate of GLP-1R agonist"** refers to a molecular complex comprising a GLP-1R agonist and one or more pharmaceutically acceptable solvent molecules. **"Hydrate of GLP-1R agonist"** refers to a molecular complex comprising a GLP-1R agonist and one or more pharmaceutically acceptable solvent molecules, wherein the solvent is water.
**"Subject"** or **"patient"** refers to an animal, in particular a mammal. In one embodiment, "subject" refers to an animal selected from the group consisting of a dog, a cat, a horse, a cow, a sheep, a goat and a non-human primate. In one preferred embodiment, "subject" refers to a human (man or woman). According to a preferred embodiment, "subject" refers to a human over the age of 18, preferably over the age of 50, more preferably over the age of 65.
**"Therapeutically effective amount"** of an active agent refers to a nontoxic but sufficient amount of said active agent to provide the desired therapeutic effect.
**"Treating"** or **"treatment"** refers to any action which makes it possible to prevent, delay, reduce in severity and/or frequency or suppress at least one symptom associated with a pathological condition, or to prevent, slow down or suppress the underlying cause of a pathological condition, or the improvement or remediation of damage. In particular, in the context of the present invention, the term **"treating"** or **"treatment"** refers more particularly to the inhibition or the slowing down of the arthritic destruction of cartilage. In one embodiment, **"treatment"** refers to a curative treatment. In another embodiment, **"treatment"** refers to a preventive treatment. In another embodiment, **"treatment"** refers to a preventive and/or curative treatment.
**"Water for injection"** is a water intended either for the preparation of parenteral drug with an aqueous vehicle (bulk water for injection) or for the dissolution or dilution of active agents or preparations for parenteral administration (sterilized water for injection).

### DETAILED DESCRIPTION

### Pharmaceutical composition

This invention relates to a pharmaceutical composition comprising at least one GLP-1R agonist or a pharmaceutically acceptable ester, salt, complex, polymorph, hydrate, solvate, enantiomer or racemate thereof, a buffer and at least one isotonic agent.

As mentioned in the above definitions, the mention of a GLP-1R agonist in the present invention also encompasses any pharmaceutically acceptable ester, salt, complex, polymorph, hydrate, solvate, enantiomer or racemate of said GLP1-R agonist.

The pharmaceutical composition of the invention comprises a GLP1-R agonist as active agent, and a mixture of excipients preferably suitable for intraarticular injection, said mixture of excipients comprising a buffer and at least one isotonic agent.

### Detailed compounds of the pharmaceutical composition

In one embodiment, the GLP-1R agonist is selected from the group consisting of a polypeptide, an antibody, a nucleic acid, an aptamer, and a small molecule. Preferably, the GLP-1R agonist is a polypeptide. The polypeptide may be selected from the group consisting of liraglutide, exenatide, lixisenatide, albiglutide, beinaglutide, dulaglutide, semaglutide, and taspoglutide. Preferably, the GLP-1R agonist is liraglutide.

In one embodiment, the buffer is selected from the group consisting of a tromethamine buffer, a phosphate buffer and any combination thereof.

In one embodiment, the buffer is a tromethamine buffer. The tromethamine buffer may comprise a buffering agent selected from the group consisting of tromethamine (Tris), tromethamine (Tris) acetate, tromethamine (Tris) phosphate, and any combination thereof. More preferably, the buffer is a tromethamine buffer comprising tromethamine (Tris) as buffering agent.

In one embodiment, the buffer is a phosphate buffer. The phosphate buffer may comprise a buffering agent selected from the group consisting of dibasic calcium phosphate, tribasic calcium phosphate, monobasic potassium phosphate, dibasic potassium phosphate, monobasic sodium phosphate, disodium phosphate and any hydrate or combination thereof. More preferably, the buffer is a phosphate buffer comprising disodium phosphate or disodium phosphate dihydrate as buffering agent. Even more preferably, the buffer is phosphate-buffered saline (PBS).

In one embodiment, the isotonic agent is selected from the group consisting of glucose, a polyethylene glycol, propylene glycol, glycerol and any combination thereof.

In one embodiment, the isotonic agent is selected from the group consisting of glucose, a polyethylene glycol, glycerol and any combination thereof.

In one embodiment, the isotonic agent is propylene glycol.

In one embodiment, the isotonic agent is a polyethylene glycol. The polyethylene glycol may be a polyethylene glycol having a molecular weight less than 800 g.mol⁻¹, preferably from 100 g.mol⁻¹ to 800 g.mol⁻¹, more preferably from 100 g.mol⁻¹ to 600 g.mol⁻¹, even more preferably a polyethylene glycol having a molecular weight from 200 g.mol⁻¹ to 400 g.mol⁻¹, even more preferably a polyethylene glycol having a molecular weight of 400 g.mol⁻¹ (=PEG400) or of 200 g.mol⁻¹ (=PEG200). In one embodiment, the polyethylene glycol is a polyethylene glycol having a molecular weight of 100, 200, 300, 400, 500, 600, 700 or 800 g.mol⁻¹.

In one embodiment, the pharmaceutical composition comprises:
- a GLP-1R agonist,
- a buffer selected from the group consisting of a tromethamine buffer and a phosphate buffer, and
- an isotonic agent selected from the group consisting of glucose, a polyethylene glycol, propylene glycol and glycerol.

In one preferred embodiment, the pharmaceutical composition comprises:
- liraglutide,
- a buffer selected from the group consisting of a tromethamine buffer and a phosphate buffer, and
- an isotonic agent selected from the group consisting of glucose, a polyethylene glycol, propylene glycol and glycerol.

In one preferred embodiment, the pharmaceutical composition comprises:
- liraglutide,
- a tromethamine buffer, and
- an isotonic agent selected from the group consisting of glucose, a polyethylene glycol, propylene glycol and glycerol.

In one preferred embodiment, the pharmaceutical composition comprises:
- liraglutide,
- a phosphate buffer, and
- an isotonic agent selected from the group consisting of glucose, a polyethylene glycol, propylene glycol and glycerol.

In one embodiment, the pharmaceutical composition comprises:
- a GLP-1R agonist,
- a buffer selected from the group consisting of a tromethamine buffer and a phosphate buffer, and
- an isotonic agent selected from the group consisting of glucose, a polyethylene glycol and glycerol.

In one preferred embodiment, the pharmaceutical composition comprises:
- liraglutide,
- a buffer selected from the group consisting of a tromethamine buffer and a phosphate buffer, and
- an isotonic agent selected from the group consisting of glucose, a polyethylene glycol and glycerol.

In one preferred embodiment, the pharmaceutical composition comprises:
- liraglutide,
- a tromethamine buffer, and
- an isotonic agent selected from the group consisting of glucose, a polyethylene glycol and glycerol.

In one preferred embodiment, the pharmaceutical composition comprises:
- liraglutide,
- a phosphate buffer, and
- an isotonic agent selected from the group consisting of glucose, a polyethylene glycol and glycerol.

In one preferred embodiment, the pharmaceutical composition comprises:
- liraglutide,
- a buffer selected from the group consisting of a tromethamine buffer and a phosphate buffer, and
- glucose as isotonic agent.

In one preferred embodiment, the pharmaceutical composition comprises:
- liraglutide,
- a buffer selected from the group consisting of a tromethamine buffer and a phosphate buffer, and
- a polyethylene glycol, preferably PEG400, as isotonic agent.

In one preferred embodiment, the pharmaceutical composition comprises:
- liraglutide,
- a buffer selected from the group consisting of a tromethamine buffer and a phosphate buffer, and
- glycerol as isotonic agent.

In one preferred embodiment, the pharmaceutical composition comprises:
- liraglutide,
- a buffer selected from the group consisting of a tromethamine buffer and a phosphate buffer, and
- propylene glycol as isotonic agent.

In one preferred embodiment, the pharmaceutical composition comprises:
- a GLP-1R agonist,
- a phosphate buffer, and
- propylene glycol as isotonic agent.

In one preferred embodiment, the pharmaceutical composition comprises:
- liraglutide,
- a phosphate buffer, and
- propylene glycol as isotonic agent.

In one preferred embodiment, the pharmaceutical composition comprises:
- liraglutide,
- a phosphate buffer,
- propylene glycol as isotonic agent, and
- phenol.

According to an embodiment, the pharmaceutical composition of the invention further comprises at least one additional excipient. The additional excipient may be a preservative. The preservative may be selected from the group consisting of phenol, cresol, resorcinol, parabens and any combination thereof. Preferably, the preservative is phenol.

According to an embodiment, the pharmaceutical composition of the invention comprises at least one further active agent used in the treatment of joint diseases, in particular of osteoarthritis. The further active agent used in the treatment of joint diseases, in particular of osteoarthritis, may be selected from the group consisting of incretins such as GIP (Glucose-dependent insulin releasing polypeptide), inhibitors of the dipeptidyl peptidase IV enzyme, growth factors or growth factors targeting agents (FGF-18, BMP7, anti-NGF agents), Wnt pathway molecules targeting agents (DYRK1A targeting agents, CLK2 targeting agents), metalloproteinases and/or aggrecanases targeting agents (ADAMTS4 targeting agents, ADAMTS5 targeting agents, MMPs targeting agents), senescence pathway targeting agents, bone resorption molecules targeting agents (cathepsin K targeting agents), analgesics (such as opioids, tramadol, acetaminophen, capsaicin), nonsteroidal anti-inflammatory drugs (such as modified angiopoietin-like 3 (ANGPTL3) protein (for example LNA043) and anti-ILl (for example anakinra, canakinumab)), steroidal anti-inflammatory drugs, symptomatic slow-acting anti-arthritic agents (SYSADOAs), hyaluronic acids, platelet rich plasmas (PRPs), alpha-1 glycoprotein and albumin.

The Glucose-dependent insulin releasing polypeptide may be selected from the group consisting of GIP receptor antagonist (for example anti-GIPR monoclonal antibody from Amgen) and tirzepatide (from Lilly).

The inhibitor of the dipeptidyl peptidase IV enzyme may be selected from the group consisting of sitagliptin, saxagliptin, vildagliptin, alogliptin and linagliptin.

The growth factors may be selected from the group consisting of fibroblast growth factor (FGF-18 sprifermin), NGF and BMP7 protein.

The growth factors targeting agents may be selected from the group consisting of fibroblast growth factor targeting agents, anti-NGF agents such as tanezumab and BMP7 protein targeting agents.

The Wnt pathway molecules targeting may be selected from the group consisting of CLK2 inhibitors, DYRK1A inhibitors and lorecivivint.

Metalloproteinases and aggrecanases may be selected from the group consisting of ADAMTS5 inhibitors and ADAMTS5 antibodies.

Senescence pathway targeting may be MDM2-p53 interaction inhibitors.

The bone resorption molecules may be cathepsin K.

The analgesics may be selected from the group consisting of acetylsalicylic acid, lysine acetylsalicylate, phenylbutazone, sulindac, diclofenac potassium or sodium, aceclofenac, tiaprofenic acid, ibuprofen, ketoprofen, alminoprofen, fenoprofen, naproxen, flurbiprofen, indomethacin, mefenamic acid, niflumic acid, tenoxicam, meloxicam, piroxicam, celecoxib, etoricoxib, betamethasone, dexamethasone, prednisone, prednisolone, tixocortol, triamcinolone, CNTX-4975 and bedinvetmab.

The nonsteroidal anti-inflammatory drugs may be selected from the group consisting of acetylsalicylic acid, lysine acetylsalicylate, phenylbutazone, sulindac, diclofenac potassium or sodium, aceclofenac, tiaprofenic acid, ibuprofen, ketoprofen, alminoprofen, fenoprofen, naproxen, flurbiprofen, indomethacin, mefenamic acid, niflumic acid, tenoxicam, meloxicam, piroxicam, celecoxib and etoricoxib.

The steroidal anti-inflammatory drugs may be selected from the group consisting of betamethasone, dexamethasone, prednisone, prednisolone, tixocortol and triamcinolone.

The symptomatic slow-acting anti-arthritic agents may be selected from the group consisting of chondroitin, chondroitin sulphate, glucosamine, glucosamine sulphate, diacerein, and unsaponifiable extracts of avocado and soya (such as in the marketed product Piascledine^{®}).

According to an embodiment, the further active agent used in the treatment of joint diseases is selected from the group consisting of hyaluronic acid, albumin and alpha-1 glycoprotein.

In one embodiment, the pharmaceutical composition is a solution. The solution may be an aqueous solution. The aqueous solution may be an aqueous solution for injection, even more particularly an aqueous solution for intra-articular injection.

In one embodiment, the pharmaceutical composition is a suspension. The solution may be an aqueous suspension. The aqueous suspension may be an aqueous suspension for injection, even more particularly an aqueous suspension for intra-articular injection.

### Concentrations of the compounds of the pharmaceutical composition

In one embodiment, the pharmaceutical composition comprises from 2 to 20 mg/mL, preferably from 2 to 8 mg/mL, more preferably from 4 to 8 mg/mL, even more preferably about 6 mg/mL, of GLP-1R agonist. In one embodiment, the pharmaceutical composition comprises about 2, 3, 4, 5, 6, 7, 8, 9, 10, 11, 12, 13, 14, 15, 16, 17, 18, 19 or 20 mg/mL, of GLP-1R agonist.

In one embodiment, the pharmaceutical composition comprises from 2 to 20 mg/mL, preferably from 2 to 8 mg/mL, more preferably from 4 to 8 mg/mL, even more preferably about 6 mg/mL, of GLP-1R agonist, wherein the GLP-1R agonist is liraglutide. In one embodiment, the pharmaceutical composition comprises about 2, 3, 4, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7, 8, 9, 10, 11, 12, 13, 14, 15, 16, 17, 18, 19 or 20 mg/mL, of GLP-1R agonist, wherein the GLP-1R agonist is liraglutide.

In one embodiment, the pharmaceutical composition comprises from 0.1 to 10 mg/mL, more preferably from 0.5 to 1 mg/mL, even more preferably about 0.97 mg/mL, of tromethamine. In one embodiment, the pharmaceutical composition comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, or 10.0 mg/mL, of tromethamine.

In one embodiment, the pharmaceutical composition comprises from 0.80 to 80 Mm, preferably from 2 to 20 mM, more preferably from 6 to 10 mM, even more preferably about 8 mM, of tromethamine.

In one embodiment, the pharmaceutical composition comprises from 0.1 to 10 mg/mL, preferably from 0.75 to 1.5 mg/mL, more preferably about 1.14 mg/mL, of disodium phosphate. In one embodiment, the pharmaceutical composition comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, or 10.0 mg/mL, of disodium phosphate. In one embodiment, the pharmaceutical composition comprises from 0.70 to 70 mM, preferably from 2 to 20 mM, more preferably from 3 to 10 mM, even more preferably about 8 mM or about 3 mM, of disodium phosphate.

In one embodiment, the pharmaceutical composition comprises from 0.05 to 10 mg/mL, preferably from 0.25 to 2.0 mg/mL, of disodium phosphate dihydrate. More preferably, the pharmaceutical composition comprises about 0.47 mg/mL of disodium phosphate dihydrate. More preferably, the pharmaceutical composition comprises about 1.42 mg/mL of disodium phosphate dihydrate. In one embodiment, the pharmaceutical composition comprises about 0.05, 0.1, 0.2, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.6, 0.7, 0.8, 0.9, 1.0, 1.1, 1.2, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49 1.50, 1.51, 1.52, 1.53, 1.54, 1.55, 1.56, 1.57, 1.58, 1.59, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, or 10.0 mg/mL, of disodium phosphate dihydrate.

In one embodiment, the pharmaceutical composition comprises from 10 to 50 mg/mL, preferably from 20 to 40 mg/mL, more preferably about 30 mg/mL, of glucose. In one embodiment, the pharmaceutical composition comprises about 10, 15, 20, 25, 26, 27, 28, 29, 30, 31, 32, 33, 34, 35, 40, 45 or 50 mg/mL, of glucose.

In one embodiment, the pharmaceutical composition comprises from 20 to 100 mg/mL, preferably from 40 to 80 mg/mL, more preferably about 60 mg/mL, of a polyethylene glycol, particularly of PEG400 or PEG200, more particularly of PEG400. In one embodiment, the pharmaceutical composition comprises about 20, 25, 30, 35, 40, 45, 50, 55, 56, 57, 58, 59, 60, 61, 62, 63, 64, 65, 70, 75, 80, 85, 90, 95 or 100 mg/mL, of a polyethylene glycol, particularly of PEG400 or PEG200, more particularly of PEG400.

In one embodiment, the pharmaceutical composition comprises from 5 to 50 mg/mL, preferably from 10 to 25 mg/mL, more preferably about 17 mg/mL or about 18 mg/mL, of glycerol. In one embodiment, the pharmaceutical composition comprises about 5, 10, 11, 12, 13, 14, 15, 16, 17, 18, 19, 20, 21, 22, 23, 24, 25, 26, 27, 28, 29, 30, 35, 40, 45 or 50 mg/mL, of glycerol.

In one embodiment, the pharmaceutical composition comprises from 1 to 20 mg/mL of propylene glycol. Preferably, the pharmaceutical composition comprises from 1 to 10 mg/mL, more preferably from 4 to 6 mg/mL, even more preferably about 4.7 mg/mL, of propylene glycol. Preferably, the pharmaceutical composition comprises from 10 to 20 mg/mL, more preferably from 12 to 16 mg/mL, even more preferably about 14 mg/mL, of propylene glycol. In one embodiment, the pharmaceutical composition comprises about 1, 2, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7, 8, 9, 10, 11, 12.0, 12.5, 13.0, 13.1, 13.2, 13.3, 13.4, 13.5, 13.6, 13.7, 13.8, 13.9, 14.0, 14.1, 14.2, 14.3, 14.4, 14.5, 14.6, 14.7, 14.8, 14.9, 15.0, 15.5, 16, 17, 18, 19 or 20 mg/mL, of propylene glycol.

In one embodiment, the pharmaceutical composition comprises from 0.1 to 10 mg/mL, preferably from 1 to 7.5 mg/mL, of phenol. Preferably, the pharmaceutical composition comprises from 1 to 2.5 mg/mL, more preferably about 1.8 mg/mL, of phenol. Preferably, the pharmaceutical composition comprises from 2.5 to 7.5 mg/mL, more preferably about 5.5 mg/mL, of phenol. In one embodiment, the pharmaceutical composition comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 7.0, 7.5, 8.0, 8.5, 9.0, 9.5, or 10.0 mg/mL, of phenol.

According to one embodiment, the pharmaceutical composition comprises or consists of:
- from 2 to 20 mg/mL, preferably from 4 to 8 mg/mL, more preferably about 6 mg/mL, of liraglutide,
- from 0.1 to 10 mg/mL, preferably from 0.75 to 1,5 mg/mL, more preferably about 1,14 mg/mL, of disodium phosphate,
- from 10 to 50 mg/mL, preferably from 20 to 40 mg/mL, more preferably about 30 mg/mL, of glucose, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.1 to 10 mg/mL, preferably from 0.5 to 1 mg/mL, more preferably about 0.97 mg/mL, of tromethamine,
- from 10 to 50 mg/mL, preferably from 20 to 40 mg/mL, more preferably about 30 mg/mL, of glucose, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.1 to 10 mg/mL, preferably from 0.75 to 1,5 mg/mL, more preferably about 1,14 mg/mL, of disodium phosphate,
- from 20 to 100 mg/mL, preferably from 40 to 80 mg/mL, more preferably about 60 mg/mL, of PEG400, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.1 to 10 mg/mL, preferably from 0.5 to 1 mg/mL, more preferably about 0.97 mg/mL, of tromethamine,
- from 20 to 100 mg/mL, preferably from 40 to 80 mg/mL, more preferably about 60 mg/mL, of PEG400, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.1 to 10 mg/mL, preferably from 0.75 to 1,5 mg/mL, more preferably about 1,14 mg/mL, of disodium phosphate,
- from 5 to 50 mg/mL, preferably from 10 to 25 mg/mL, more preferably about 17 mg/mL, of glycerol, and
- water for injection.

According to one embodiment, the pharmaceutical composition comprises or consists of:
- from 2 to 20 mg/mL, preferably from 4 to 8 mg/mL, more preferably about 6 mg/mL of liraglutide,
- from 0.1 to 10 mg/mL, preferably from 0.5 to 1 mg/mL, more preferably about 0.97 mg/mL, of tromethamine,
- from 5 to 50 mg/mL, preferably from 10 to 25 mg/mL, more preferably about 18 mg/mL, of glycerol, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.05 to 10 mg/mL, preferably from 0.25 to 2.0 mg/mL more preferably about 0.47 mg/mL, of disodium phosphate dihydrate,
- from 1 to 20 mg/mL, preferably from 4 to 6 mg/mL, more preferably about 4.7 mg/mL, of propylene glycol, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.05 to 10 mg/mL, preferably from 0.25 to 2.0 mg/mL, more preferably about 0.47 mg/mL, of disodium phosphate dihydrate,
- from 1 to 20 mg/mL, preferably from 4 to 6 mg/mL, more preferably about 4.7 mg/mL, of propylene glycol,
- from 0.1 to 10 mg/mL, preferably from 1 to 2.5 mg/mL, more preferably about 1.8 mg/mL, of phenol, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.05 to 10 mg/mL, preferably from 0.25 to 2.0 mg/mL, more preferably about 1.42 mg/mL, of disodium phosphate dihydrate,
- from 1 to 20 mg/mL, preferably from 12 to 16 mg/mL, more preferably about 14 mg/mL, of propylene glycol, and
- water for injection.

According to one embodiment, the pharmaceutical composition comprises or consists of:
- from 2 to 20 mg/mL, preferably from 4 to 8 mg/mL, more preferably about 6 mg/mL, of liraglutide,
- from 0.05 to 10 mg/mL, preferably from 0.25 to 2.0 mg/mL, more preferably about 1.42 mg/mL, of disodium phosphate dihydrate,
- from 1 to 20 mg/mL, preferably from 12 to 16 mg/mL, of propylene glycol,
- from 0.1 to 10 mg/mL, preferably from 2.5 to 7.5 mg/mL, more preferably about 5.5 mg/mL, of phenol, and
- water for injection.

The inventors have surprisingly found that the pharmaceutical compositions according to the invention as described above induce a long-term effect when administered via intraarticular injection, in particular an effect that lasts at least three weeks.

### Pharmaceutical composition for use for treating at least one joint disease

The present invention also relates to a method of treating at least one joint disease by administering to a patient in need thereof an effective amount of a pharmaceutical composition according to the invention as described above.

The present invention also relates to the use of a pharmaceutical composition according to the invention as described above for the manufacture of a medicament for the treatment of at least one joint disease.

The present invention also relates to the use of a pharmaceutical composition according to the invention as described above for the treatment of at least one joint disease.

The present invention also relates to a pharmaceutical composition according to the invention for use in a method for treating at least one joint disease.

### Joint diseases

In one embodiment, said at least one joint disease is a chronic disease. The chronic disease may be osteoarthritis.

In one embodiment, said at least one joint disease is an acute disease. The acute disease may be an acute pain.

In one embodiment, said at least one joint disease is selected from the group consisting of osteoarthritis, joint pain and their combination. The joint pain may be inflammatory joint pain or non-inflammatory joint pain.

The joint disease to be treated, in particular the osteoarthritis, may affect any joint, for example the joints of the hip (coxarthrosis), of the knee (gonarthrosis), of the ankle, of the foot, of the hand, of the wrist, of the elbow, of the shoulder and/or of the rachis. Preferably, the joint disease to be treated, in particular the osteoarthritis, affects the joints of the hip, of the knee, of the hand and/or of the rachis.

Treating at least one joint disease may comprise reducing an existing joint inflammation in a subject in need thereof.

Treating at least one joint disease may comprise increasing the chondrocyte proliferation and/or chondrocyte differentiation and/or decreasing synovitis in a subject in need thereof.

### Administration

In one embodiment, the pharmaceutical composition is administered or is to be administered via intraarticular injection, in particular via intraarticular injection into the joint cavity.

The pharmaceutical composition may be administered in combination with at least one other locally acting substances such as hyaluronic acid, nonsteroidal anti-inflammatory drugs, stem cells, growth factors (such as sprifermine or BMP7), MMPs inhibitors, Wnt inhibitors and/or analgesic substances.

According to an embodiment, the pharmaceutical composition is administered or is to be administered at a dose from 0.7 µg to 180 µg of liraglutide, preferably from 20 µg to 180 µg, more preferably from 20 µg to 120 µg, of liraglutide. One dose refers to the cumulative amount of GLP-1R agonists administered in 2 weeks to 1 month. In one embodiment, the pharmaceutical composition is administered or is to be administered at a dose of 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179 or 180 µg, of liraglutide.

According to an embodiment, the pharmaceutical composition is administered or is to be administered at a dose from 0.0245 mg to 6.3 mg of liraglutide, preferably from 0.7 mg to 6.3 mg, of liraglutide. One dose refers to the cumulative amount of GLP-1R agonists administered in 2 weeks to 1 month. In one embodiment, the pharmaceutical composition is administered or is to be administered at a dose of 0.0245, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28, 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, 1.93, 1.94, 1.95, 1.96, 1.97, 1.98, 1.99, 2.00, 2.05, 2.10, 2.15, 2.20, 2.25, 2.30, 2.35, 2.40, 2.45, 2.50, 2.55, 2.60, 2.65, 2.70, 2.75, 2.80, 2.85, 2.90, 2.95, 3.00, 3.05, 3.10, 3.15, 3.20, 3.25, 3.30, 3.35, 3.40, 3.45, 3.50, 3.55, 3.60, 3.65, 3.70, 3.75, 3.80, 3.85, 3.90, 3.95, 4.00, 4.05, 4.10, 4.15, 4.20, 4.25, 4.30, 4.35, 4.40, 4.45, 4.50, 4.55, 4.60, 4.65, 4.70, 4.75, 4.80, 4.85, 4.90, 4.95, 5.00, 5.05, 5.10, 5.15, 5.20, 5.25, 5.30, 5.35, 5.40, 5.45, 5.50, 5.55, 5.60, 5.65, 5.70, 5.75, 5.80, 5.85, 5.90, 5.95, 6.00, 6.05, 6.10, 6.15, 6.20, 6.25 or 6.3 mg, of liraglutide.

In one embodiment, said dose of said pharmaceutical composition is administered or is to be administered in one or at least two intraarticular injections.

In a preferred embodiment, said dose of said pharmaceutical composition is administered or is to be administered in one intraarticular injection. For example, the dose is 20 µg of liraglutide and is administered or is to be administered in one intraarticular injection. This intraarticular injection may be repeated every two weeks. This intraarticular injection may be repeated every three weeks. This intraarticular injection may be repeated every month.

In another preferred embodiment, said dose of said pharmaceutical composition is administered or is to be administered in two intraarticular injections, for example both intraarticular injections are administered or are to be administered on the same day or both injections are administered or are to be administered on different days. In particular, the dose of the pharmaceutical composition may be administered in two intraarticular injections: for example, the first at J1 and the second at J8 or the first at J1 and the second at J15.

When the dose is administered or is to be administered in multiple injections, each injection may contain the same amount of GLP-1R agonist or varying amounts of GLP-1R agonist. For example, the dose is 20 µg of liraglutide and is administered or is to be administered in two intraarticular injections of 10 µg of liraglutide: the first injection at J1 and the second injection at J8, or the first injection at J1 and the second injection at J15; these two injections may be repeated every three weeks, that is that the further first injection is at J22. These two injections may also be repeated every month, that is that the further first injection is at J29. In the case were the first injection is at J1 and the second injection is at J8, two injections may also be repeated every two weeks, that is that the further first injection is at J15.

Indeed, the inventors of the present invention have surprisingly discovered that two intraarticular injections of a half-dose of liraglutide (one injection at J1 and one injection at J8) induce the same analgesic effect and the same duration of analgesic effect as one intraarticular injection of the dose of liraglutide (at J1). For example, two intraarticular injections of 10 µg of liraglutide (one injection at J1 and one injection at J8) induce the same analgesic effect and the same duration of analgesic effect as one intraarticular injection of 20 µg of liraglutide (at J1).

In one embodiment, several doses of the pharmaceutical composition are administered or are to be administered to the subject, the doses being administered every two weeks to every month. In one preferred embodiment, several doses of the pharmaceutical composition are administered or are to be administered to the subject, the doses being administered every three weeks. In one more preferred embodiment, several doses of the pharmaceutical composition are administered or are to be administered to the subject, the doses being administered every month.

Indeed, the inventors of the present invention have surprisingly discovered that the pharmaceutical compositions according to the invention have a three weeks long-lasting analgesic effect following acute intraarticular administration.

### BRIEF DESCRIPTION OF THE DRAWINGS

Figure 1: IC₅₀ determination on NO secretion by different concentrations of Liraglutide pre-formulation 1 ranging from 4.1nM to 3µM in LPS-stimulated conditions (nM). Mean ± SEM, n=4 (each condition performed in quadruplicates)
Figure 2: IC₅₀ determination on NO secretion by different concentrations of Liraglutide pre-formulation 2 ranging from 4.1nM to 3µM in LPS-stimulated conditions (nM). Mean ± SEM, n=4 (each condition performed in quadruplicates).
Figure 3: IC₅₀ determination on NO secretion by different concentrations of Liraglutide pre-formulation 3 ranging from 4.1nM to 3µM in LPS-stimulated conditions (nM). Mean ± SEM, n=4 (each condition performed in quadruplicates)
Figure 4: IC₅₀ determination on NO secretion by different concentrations of Liraglutide pre-formulation 4 ranging from 4.1nM to 3µM in LPS-stimulated conditions (nM). Mean ± SEM, n=4 (each condition performed in quadruplicates)
Figure 5: IC₅₀ determination on NO secretion by different concentrations of Liraglutide pre-formulation 5 ranging from 4.1nM to 3µM in LPS-stimulated conditions (nM). Mean ± SEM, n=4 (each condition performed in quadruplicates)
Figure 6: IC₅₀ determination on NO secretion by different concentrations of Liraglutide pre-formulation 6 ranging from 4.1nM to 3µM in LPS-stimulated conditions (nM). Mean ± SEM, n=4 (each condition performed in quadruplicates)
Figure 7: IC₅₀ determination on NO secretion by different concentrations of Liraglutide pre-formulation 7 ranging from 4.1nM to 3µM in LPS-stimulated conditions (nM). Mean ± SEM, n=4 (each condition performed in quadruplicates)
Figure 8: IC₅₀ determination on NO secretion by different concentrations of Liraglutide pre-formulation 8 ranging from 4.1nM to 3µM in LPS-stimulated conditions (nM). Mean ± SEM, n=4 (each condition performed in quadruplicates)
Figure 9: IC₅₀ determination on NO secretion by different concentrations of Liraglutide pre-formulation 9 ranging from 4.1nM to 3µM in LPS-stimulated conditions (nM). Mean ± SEM, n=4 (each condition performed in quadruplicates)
Figure 10: IC₅₀ determination on NO secretion by different concentrations of Liraglutide pre-formulation 10 ranging from 4.1nM to 3µM in LPS-stimulated conditions (nM). Mean ± SEM, n=4 (each condition performed in quadruplicates)
Figure 11: IC₅₀ determination on NO secretion by different concentrations of Victoza^{®} ranging from 4.1nM to 3 µM in LPS-stimulated conditions (nM). Mean ± SEM, n=4 (each condition performed in quadruplicates)
Figure 12: IC₅₀ determination on NO secretion by different concentrations of Liraglutide pre-formulation 2 ranging from 4.1nM to 3µM in IL-1β-stimulated conditions (nM). Mean ± SEM, n=4 (each condition performed in quadruplicates)
Figure 13: IC₅₀ determination on NO secretion by different concentrations of Liraglutide pre-formulation 6 ranging from 4.1nM to 3µM in IL-1β-stimulated conditions (nM). Mean ± SEM, n=4 (each condition performed in quadruplicates)
Figure 14: IC₅₀ determination on NO secretion by different concentrations of Victoza^{®} (PBS) ranging from 4.1nM to 3µM in IL-1β-stimulated conditions (nM). Mean ± SEM, n=4 (each condition performed in quadruplicates)
Figure 15: Von Frey test withdrawal threshold on right hind paw up to day 11. Mean ± SEM, n=8 per group. Figure 15A: Preformulation-2 compared to control vehicle, MIA/vehicle and Victoza (20µg) group. Figure 15B: Preformulation-6 compared to control vehicle, MIA/vehicle and Victoza (20µg) group.
   - *** p<0.001 Victoza 20µg group (3M) vs MIA/vehicle group (2M) at D7 and D10;
   - *** p<0.001 MIA/vehicle group (2M) vs Preformulation-2 and -6 at 20µg and 120µg group (9M, 8M, 6M and 5M) at D7 and D10;
   - ** p<0.001 MIA/vehicle group (2M) vs Preformulation-2 at 3.3µg group (4M) at D7;
   - *** p<0.001 MIA/vehicle group (2M) vs Preformulation-2 at 3.3µg group (4M) at D10;
   - ** p<0.01 MIA/vehicle group (2M) vs Preformulation-2 at 3.3µg group (7M) at D7; and
   - ** p<0.01 MIA/vehicle group (2M) vs Preformulation-2 at 3.3µg group (7M) at D10.

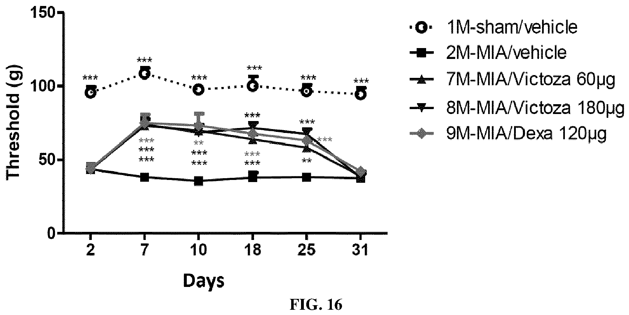

Figure 16: Von Frey test withdrawal threshold on right hind paw up to day 32. Mean ± SEM, n=8 per group. ** p<0.01, *** p<0.001 vs MIA/vehicle group 2M.

### EXAMPLES

The present invention is further illustrated by the following examples.

### Example 1: pH and osmolality of compositions according to the present invention

Six pharmaceutical compositions were prepared.
Pre-formulation 1 contained: 6 mg/mL of liraglutide, 8 mM of disodium phosphate, 30 mg/mL of glucose and water for injection.
Pre-formulation 2 contained: 6 mg/mL of liraglutide, 8 mM of tromethamine, 30 mg/mL of glucose and water for injection.
Pre-formulation 5 contained: 6 mg/mL of liraglutide, 8 mM of disodium phosphate, 60 mg/mL of PEG400 and water for injection.
Pre-formulation 6 contained: 6 mg/mL of liraglutide, 8 mM of tromethamine, 60 mg/mL of PEG400 and water for injection.
Pre-formulation 7 contained: 6 mg/mL of liraglutide, 8 mM of disodium phosphate, 17 mg/mL of glycerol and water for injection.
Pre-formulation 8 contained: 6 mg/mL of liraglutide, 8 mM of tromethamine, 18 mg/mL of glycerol and water for injection.

The pH and the osmolality of the compositions were measured:

**Table 1**

| | pH | Osmolality (mmol/kg) | | |
|---|---|---|---|---|
| | | T0 | T0 + 48 hours | T0 + 1 month |
| Preformulation 1 | 8.2 | 207 | 207 | 210 |
| Preformulation 2 | 8.6 | 209 | 211 | 215 |
| Preformulation 5 | 8.4 | 202 | 210 | 220 |
| Preformulation 6 | 8.6 | 198 | 203 | 216 |
| Preformulation 7 | 8.4 | 202 | 206 | 212 |
| Preformulation 8 | 8.6 | 181 | 189 | 199 |

The results of osmolality and particle size measurements, are presented in the following table:

**Table 2**

| | | | **Osmolality (mmol/kg)** | | | **Particles size (nm)** | | |
|---|---|---|---|---|---|---|---|---|
| **Pre-formulation** | **Composition** | **pH** | ***T0*** | ***T*+*48h*** | **T+1Mo** | ***T0*** | ***T*+*48h*** | **T+1Mo** |
| 1 | Glucose + Phosphate | 8.2 | *207* | *207* | 210 | *110* | *70* | 234 |
| 2 | Glucose + Tromethamine | 8.6 | *209* | *211* | 215 | *313* | *227* | 100 |
| 5 | PEG400 + Phosphate | 8.4 | *202* | *210* | 220 | *129* | *37* | 110 |
| 6 | PEG400 + Tromethamine | 8.6 | *198* | *203* | 216 | *329* | *148* | 347 |
| 7 | Glycerol + Phosphate | 8.4 | *202* | *206* | 212 | *68* | *46* | 70 |
| 8 | Glycerol + Tromethamine | 8.6 | *181* | *189* | 199 | *412* | *239* | 400 |

It appeared that the six compositions had a pH allowing the solubilization of liraglutide. Moreover, the osmolality values after 48 hours and after one month were similar to the ones obtained at T0. The compositions osmolality was therefore stable.

### Example 2: In vitro efficacy of 10 compositions according to the invention on murine macrophages cell line RAW 264.7

### Test system

The cell line used is RAW 264.7 (Macrophages from Balb/C male mice transformed by Abelson murine leukemia virus), which ATCC reference is TIB-71.

### Test items and test items vehicle

**Table 3**

| Pre-formulation name | Isotonic agent | Buffer agent | Liraglutide API (6mg/ml) |
|---|---|---|---|
| Pre-formulation 1 | Glucose | Disodium phosphate (Na₂HPO₄) (8 mM) | yes |
| Pre-formulation 2 | Glucose | Tromethamine | yes |
| Pre-formulation 3 | Propylene glycol | Tromethamine | yes |
| Pre-formulation 4 | - | Disodium phosphate and monosodium phosphate (Na₂HPO₄/NaH₂PO₄) (VWR 28020.292 - 18D044123) (VWR 28011.260 - 18C224139 (≈ 130 mM, iv DRUGABILIS buffer) | yes |
| Pre-formulation 5 | PEG400 | Disodium phosphate (Na₂HPO₄) (8 mM) | yes |
| Pre-formulation 6 | PEG400 | Tromethamine | yes |
| Pre-formulation 7 | Glycerol | Disodium phosphate (Na₂HPO₄) (8 mM) | yes |
| Pre-formulation 8 | Glycerol | Tromethamine | yes |
| Pre-formulation 9 | - | Dulbecco's Phosphate Buffered Saline | yes |
| Pre-formulation 10 | Propylene glycol | Disodium phosphate (Na₂HPO₄) (8 mM) | yes |

Pre-formulations 1, 2, 5, 6, 7 and 8 of example 2 are identical to pre-formulations respectively 1, 2, 5, 6, 7 and 8 of example 1.

### Formulation

Except when another reference is indicated, for the ten pre-formulations, the glucose is Sigma 16325 - SZBF2860V, the disodium phosphate is Sigma - 04276 - 90900, the tromethamine is Sigma T6687 - WXBC2569V, the Dulbecco's Phosphate Buffered Saline is Sigma D8662 -RNBJ0600, the propylene glycol is Sigma 16033 - SZBC2900V, the PEG400 is Sigma 81172 - BCBT2825, and the glycerol is Fluka 49783 - BCBD0423.

LPS (Sigma) supplied as a powder was prepared at 1mg/ml in PBS. The concentrated stock solution was diluted to the final concentration of 100ng/ml in DMEM treatment medium for experiment.

Pre-formulations of Liraglutide (1 to 10) supplied as solutions at 6mg/ml of liraglutide were diluted in PBS as a stock solution. The concentrated stock solution was then diluted in DMEM treatment medium to reach the final concentrations of 3µM, 1µM, 333.3nM, 111.1nM, 37.0nM, 12.3nM and 4.1nM.

Victoza^{®} (Novo nordisk) is a commercial Liraglutide drug. Test item supplied as a solution at 6mg/ml was diluted in PBS as a stock solution. The concentrated stock solution was then diluted in DMEM treatment medium to reach the final concentrations of 3µM, 1µM, 333.3nM, 111.1nM, 37.0nM, 12.3nM and 4.1nM.

The vehicle (PBS) is supplied "ready to use" and was diluted in cell treatment medium at a final concentration of 1:100.

### EXPERIMENTAL DESIGN

### Cell culture

The cells were cultured until 80% confluence and harvested using a cell scraper and resuspended in a new flask; until enough cells are obtained to start the study.

### Seeding

The cells were harvested at 70-80% confluence using a cell scraper, counted and re-suspended to a final concentration of 1x10⁶ cells/ml in seeding medium (DMEM High glucose + 1% P/S). Next, the cells were seeded in 96-well microtiter plate (100µl/well - 100 000 cells/well). The cell cultures were maintained under sterile condition in an incubator for 24h at 37°C with 5% CO₂.

### Treatment

The seeding medium was aspirated from the 96-microtiter plate. Then, 200µl of medium containing vehicle or LPS at 100ng/ml with different doses of pre-formulated Liraglutide or Victoza^{®} (4.1nM to 3µM) or vehicles were added to each well according to **Table 4** for study design and **Table 5** for study timeline. The plates were incubated 24 hours at 37°C with 5% CO₂.

**Table 4 - Study design**

| **Group** | **Treatment** | **Induction** | **Time** |
|---|---|---|---|
| 1 | Cells only (Blank) | Vehicle (PBS) | 24 hours co-treatment |
| 2 | Vehicle (PBS) | LPS 100ng/ml | |
| 3 | Pre-formulation 14.1nM | | |
| 4 | Pre-formulation 1 12.3nM | | |
| 5 | Pre-formulation 1 37.0nM | | |
| 6 | Pre-formulation 1 111.1nM | | |
| 7 | Pre-formulation 1 333.3nM | | |
| 8 | Pre-formulation 1 1µM | | |
| 9 | Pre-formulation 1 3µM | | |
| 10 | Pre-formulation 2 4.1nM | | |
| 11 | Pre-formulation 2 12.3nM | | |
| 12 | Pre-formulation 2 37.0nM | | |
| 13 | Pre-formulation 2 111.1nM | | |
| 14 | Pre-formulation 2 333.3nM | | |
| 15 | Pre-formulation 2 1µM | | |
| 16 | Pre-formulation 2 3µM | | |
| 17 | Pre-formulation 3 4.1nM | | |
| 18 | Pre-formulation 3 12.3nM | | |
| 19 | Pre-formulation 3 37.0nM | | |
| 20 | Pre-formulation 3 111.1nM | | |
| 21 | Pre-formulation 3 333.3nM | | |
| 22 | Pre-formulation 3 1µM | | |
| 23 | Pre-formulation 3 3µM | | |
| 24 | Pre-formulation 4 4.1nM | | |
| 25 | Pre-formulation 4 12.3nM | | |
| 26 | Pre-formulation 4 37.0nM | | |
| 27 | Pre-formulation 4 111.1nM | | |
| 28 | Pre-formulation 4 333.3nM | | |
| 29 | Pre-formulation 4 1µM | | |
| 30 | Pre-formulation 4 3µM | | |
| 31 | Pre-formulation 5 4.1nM | | |
| 32 | Pre-formulation 5 12.3nM | | |
| 33 | Pre-formulation 5 37.0nM | | |
| 34 | Pre-formulation 5 111.1nM | | |
| 35 | Pre-formulation 5 333.3nM | | |
| 36 | Pre-formulation 5 1µM | | |
| 37 | Pre-formulation 5 3µM | | |
| 38 | Pre-formulation 64.1nM | | |
| 39 | Pre-formulation 6 12.3nM | | |
| 40 | Pre-formulation 6 37.0nM | | |
| 41 | Pre-formulation 6 111.1nM | | |
| 42 | Pre-formulation 6 333.3nM | | |
| 43 | Pre-formulation 6 1µM | | |
| 44 | Pre-formulation 6 3µM | | |
| 45 | Pre-formulation 7 4.1nM | | |
| 46 | Pre-formulation 7 12.3nM | | |
| 47 | Pre-formulation 7 37.0nM | | |
| 48 | Pre-formulation 7 111.1nM | | |
| 49 | Pre-formulation 7 333.3nM | | |
| 50 | Pre-formulation 7 1µM | | |
| 51 | Pre-formulation 7 3µM | | |
| 52 | Pre-formulation 84.1nM | | |
| 53 | Pre-formulation 8 12.3nM | | |
| 54 | Pre-formulation 8 37.0nM | | |
| 55 | Pre-formulation 8 111.1nM | | |
| 56 | Pre-formulation 8 333.3nM | | |
| 57 | Pre-formulation 8 1µM | | |
| 58 | Pre-formulation 8 3µM | | |
| 59 | Pre-formulation 94.1nM | | |
| 60 | Pre-formulation 9 12.3nM | | |
| 61 | Pre-formulation 9 37.0nM | | |
| 62 | Pre-formulation 9 111.1nM | | |
| 63 | Pre-formulation 9 333.3nM | | |
| 64 | Pre-formulation 9 1µM | | |
| 65 | Pre-formulation 9 3µM | | |
| 66 | Pre-formulation 10 4.1nM | | |
| 67 | Pre-formulation 10 12.3nM | | |
| 68 | Pre-formulation 10 37.0nM | | |
| 69 | Pre-formulation 10 111.1nM | | |
| 70 | Pre-formulation 10 333.3nM | | |
| 71 | Pre-formulation 10 1µM | | |
| 72 | Pre-formulation 10 3µM | | |
| 73 | Victoza^{®} 4.1nM (in PBS) | | |
| 74 | Victoza^{®} 12.3nM (in PBS) | | |
| 75 | Victoza^{®} 37.0nM (in PBS) | | |
| 76 | Victoza^{®} 111.1nM (in PBS) | | |
| 77 | Victoza^{®} 333.3nM (in PBS) | | |
| 78 | Victoza^{®} 1µM (in PBS) | | |
| 79 | Victoza^{®} 3µM (in PBS) | | |

Each condition treatment was run in quadruplicate.

**Table 5 - Study schedule**

| Procedure | Study day | | | | |
|---|---|---|---|---|---|
| | Before study | 1 | 2 | 3 | After study |
| RAW 264.7 cell culture | √ | | | | |
| Plating and starving RAW 264.7 cells | | √ | | | |
| Stimulation with LPS +/- Liraglutide or Victoza^{®} or vehicles | | | √ | | |
| Collect the supernatant | | | | √ | |
| Dosages NO | | | | | √ |

### TESTS AND EVALUATIONS

At study termination, culture medium (± 200µl) of each well was collected in 1.5ml tube (1 tube per well), centrifuged at 4000rpm at room temperature and supernatant were added to a new 1.5ml tube.

### Nitrite oxide dosage (Griess reagent)

Nitrite reagent assay was performed according to Manufacturer's instructions (Nitrite Reagent Assay, Promega). The test system is based on a chemical reaction which transform sulfanilamide in azo compound in presence of nitrite ions (NO₂⁻) and N-1-napthylethylenediamine dihydrochloride (NED). Azo compound coloration is detectable at 540nm. The solutions are "ready to use". Nitrite standard was diluted in culture medium to obtain a reference curve (0-100µM) for quantification. 50µl of blank, standard or culture supernatant were added to wells of a 96-microtiter plate. 50µl of Sulfanilamide Solution were added to the wells and the plate was incubated for 5-10 minutes in the dark. Then, 50µl of NED Solution were added to the wells and the plate was incubated for 5-10 minutes in the dark. A purple/magenta color forms immediately and the absorbance was measured within 30 minutes at 540nm. The average optical density (OD) of read blank wells was subtracted from each reading. Concentration of nitrite ions was calculated from the reference curve.

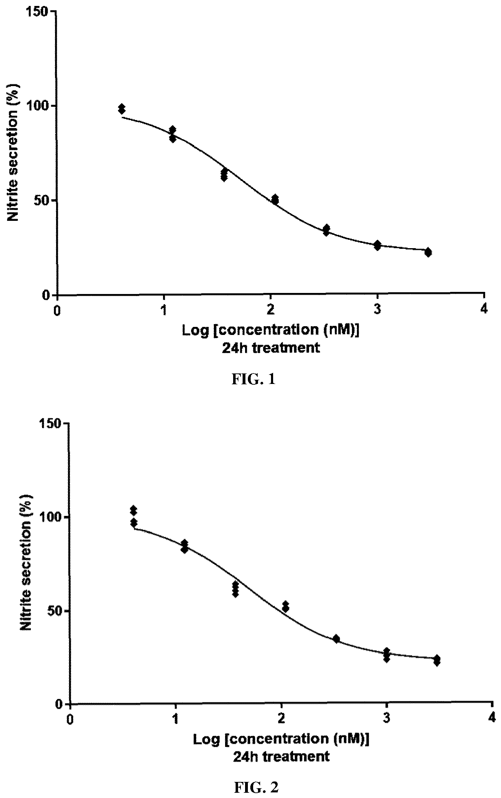

As expected, NO production in vehicle conditions was below the limit of detection and were attributed a value of 1.56µM. In LPS-stimulated conditions, detection of nitrite oxide in culture medium is significantly increased compared to the vehicle. This secretion was significantly reduced by each tested-dose of Liraglutide pre-formulations, or Victoza^{®} compared to the LPS alone condition, with a sigmoidal dose response pattern. Results are presented in Figure 1 to 11. IC₅₀ was calculated for each formulation using Prism software and summary results are presented in **Table 6.**

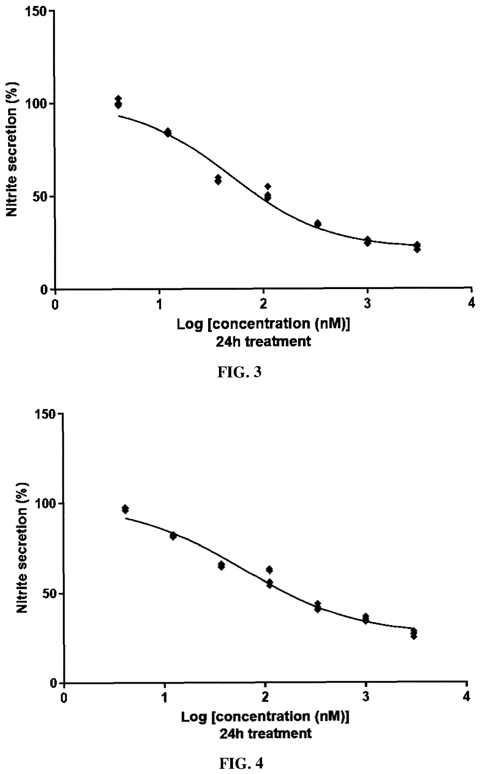

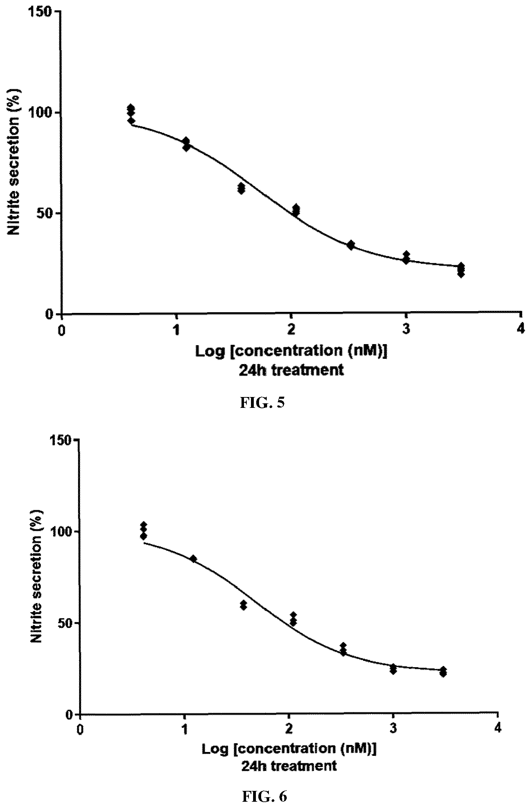

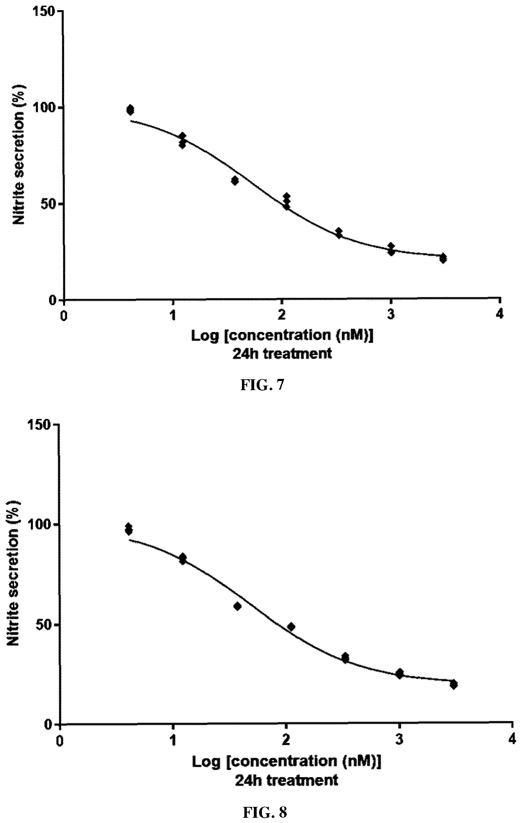

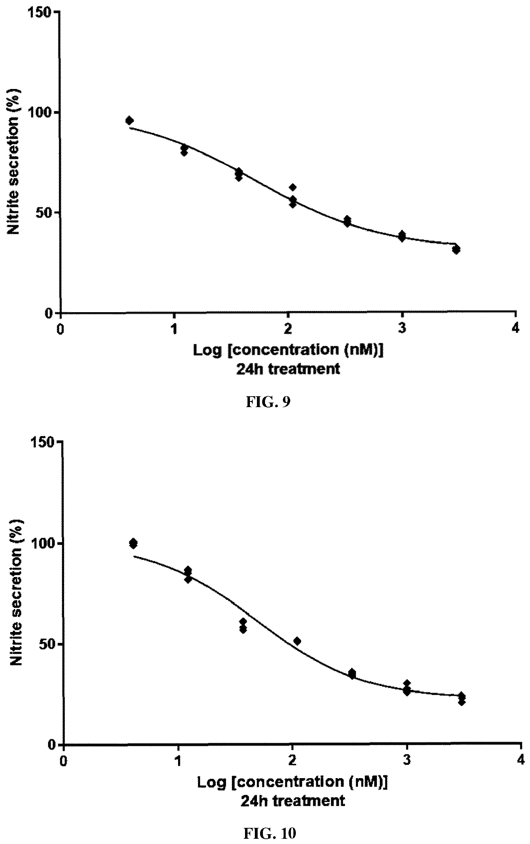

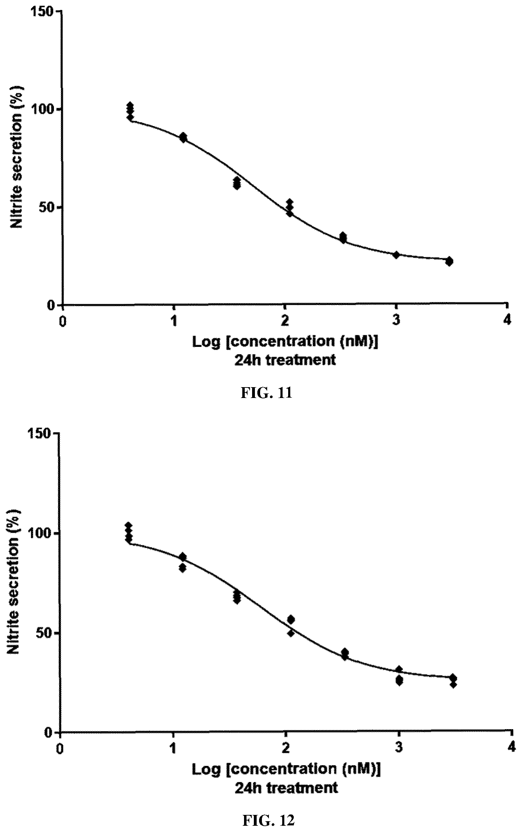

For RAW 264.7 cells cultured in high glucose conditions, Liraglutide pre-formulation 1 IC₅₀ dose on NO secretion is 53nM with a confidence interval between 48nM and 59nM (Figure 1); Liraglutide pre-formulation 2 IC₅₀ dose on NO secretion is 51nM with a confidence interval between 44nM and 59nM (Figure 2); Liraglutide pre-formulation 3 IC₅₀ dose on NO secretion is 50nM with a confidence interval between 43nM and 59nM (Figure 3); Liraglutide pre-formulation 4 IC₅₀ dose on NO secretion is 61nM with a confidence interval between 52nM and 72nM (Figure 4); Liraglutide pre-formulation 5 IC₅₀ dose on NO secretion is 53nM with a confidence interval between 47nM and 61nM (Figure 5); Liraglutide pre-formulation 6 IC₅₀ dose on NO secretion is 49nM with a confidence interval between 42nM and 57nM (Figure 6); Liraglutide pre-formulation 7 IC₅₀ dose on NO secretion is 54nM with a confidence interval between 47nM and 62nM (Figure 7); Liraglutide pre-formulation 8 IC₅₀ dose on NO secretion is 48nM with a confidence interval between 43nM and 55nM (Figure 8); Liraglutide pre-formulation 9 IC₅₀ dose on NO secretion is 53nM with a confidence interval between 47nM and 59nM (Figure 9); Liraglutide pre-formulation 10 IC₅₀ dose on NO secretion is 50nM with a confidence interval between 42nM and 58nM (Figure 10) and Victoza^{®} (PBS) IC₅₀ dose on NO secretion is 52nM with a confidence interval between 46nM and 58nM (Figure 11).

**Table 6 - IC₅₀ summary table**

| **Formulation** | **IC₅₀ value** |
|---|---|
| Victoza^{®} (PBS) | 52nM (46-58) |
| Pre-formulation 1 | 53nM (48-59) |
| Pre-formulation 2 | 51nM (44-59) |
| Pre-formulation 3 | 50nM (43-59) |
| Pre-formulation 4 | 61nM (52-72) |
| Pre-formulation 5 | 53nM (47-61) |
| Pre-formulation 6 | 49nM (42-57) |
| Pre-formulation 7 | 54nM (47-62) |
| Pre-formulation 8 | 48nM (43-55) |
| Pre-formulation 9 | 53nM (47-59) |
| Pre-formulation 10 | 50nM (42-58) |

### Conclusion

The objective of the study was to test 7 doses of 10 Liraglutide pre-formulations in comparison to Victoza^{®}, a marketed Liraglutide drug (4.1nM to 3µM) in RAW 264.7 cells, a murine macrophage cell line.

We demonstrated that Liraglutide pre-formulations or Victoza^{®} test items were able to dose-dependently inhibit the LPS-induced NO production in RAW 264.7 cells.

Liraglutide pre-formulations, Liraglutide API and Victoza^{®} IC₅₀ dose on NO production in RAW 264.7 cell line model in LPS-stimulated conditions was calculated. We confirmed that the ten tested Liraglutide pre-formulations have overall the same anti-inflammatory effect as Victoza^{®}.

The *in vitro* efficacy of pre-formulations 1 to 10 is demonstrated, with an average IC₅₀ value of 48nM to 61nM.

### Example 3: In vitro efficacy of pre-formulations 2 and 6 of example 2 on murine primary chondrocytes

### Test system

Murine primary chondrocytes derived from C57B1/6 newborn mice.

### Test items and test items vehicle

**Table 7**

| Pre-formulation name | Isotonic agent | Buffer agent | Liraglutide API (6mg/ml) |
|---|---|---|---|
| Pre-formulation 2 | Glucose | Tromethamine | yes |
| Pre-formulation 6 | PEG400 | Tromethamine | yes |

### Formulations:

Interleukine-1β (IL-1β) (PeproTech) supplied as a powder was dissolved in water as a stock solution at 0.1mg/ml. The concentrated stock solution was then diluted at final concentration (2ng/ml) into the medium containing supplements (P/S, BSA, +/-Liraglutide).

Pre-formulations of Liraglutide (2 and 6) supplied as solutions at 6mg/ml were diluted in PBS as a stock solution. The concentrated stock solution was then diluted in DMEM medium to reach the final concentrations of 3µM, 1µM, 333.3nM, 111.1nM, 37.0nM, 12.3nM and 4.1nM.

Victoza^{®} is a commercial Liraglutide drug. Test item supplied as a solution at 6mg/ml was diluted in PBS as a stock solution. The concentrated stock solution was then diluted in DMEM medium to reach the final concentrations of 3µM, 1µM, 333.3nM, 111.1nM, 37.0nM, 12.3nM and 4.1nM.

The vehicle (PBS) is supplied "ready to use" and were diluted in cell seeding medium at a final concentration of 1:100.

### EXPERIMENTAL DESIGN

### Isolation of murine articular cartilage

Immature murine chondrocytes were derived from newborn pups (5-6 days old C57B1/6). Joint was cleaned from the surrounding tissue with a scalpel, then it was cut in half to separate the two spheres, and then cut in half again. This allows for easier digestion. Femoral heads and condyles and tibial plateau were placed also in 30ml of 1X PBS.

### Isolation of immature murine chondrocytes

Pieces of cartilage were incubated twice in 10ml of digestion solution (DMEM, 2mM L-Glutamine, 1g/L of glucose + 1% P/S + Collagenase 3mg/ml) for 45min in an incubator at 37°C with 5% CO₂ in a Petri dish 100mm. Between the two digestions, pieces of cartilage were retrieved and placed in a new Petri dish. After the two digestions, dispersion of the aggregates was performed to obtained a suspension of isolated cells. Pieces of cartilage were incubated in 10ml DMEM, 2mM L-Glutamine, 1g/L of glucose + 1% P/S with collagenase D solution at 0.5mg/ml (diluted to 1/6) overnight in an incubator at 37°C with 5% CO₂.

### Seeding chondrocytes

After overnight digestion, 10ml of DMEM, 2mM L-Glutamine, 1g/L of glucose + 10% FBS + 1% P/S were added to a Petri dish to stop the collagenase D action. After dispersion of the aggregates with decreasing pipette sizes, a suspension of isolated cells was obtained and filtered through a sterile 70µm cell strainer. Then, the cells were centrifuged for 10min at 400g at 20°C. The medium was removed and the pellet was resuspended in 5ml of PBS to wash the cells. The cells were centrifuged for 10min at 400g at 20°C, and the PBS was replaced by 15ml of DMEM 2mM L-Glutamine, 1g/L of glucose + 10% FBS + 1% P/S. The chondrocytes were counted in a Neubauer hemocytometer and observed to assess the viability of extracted cells. Chondrocytes were seeded at a density of 40x10³ cells in 2 ml of DMEM 2mM L-Glutamine, 1g/L of glucose + 10% FBS + 1% P/S per well in 12-well plates. The culture was maintained under sterile conditions in an incubator at 37°C with 5% CO₂.

### Culture of chondrocytes

Immature murine articular chondrocytes are expected to reach confluence by 6-7 days of culture. The culture medium was changed after 3 days of culture. At day 7, the DMEM medium containing 10% FBS was removed, the wells were rinsed twice with 1ml of PBS and 1ml of DMEM, 2mM L-Glutamine, 1g/L of glucose + 1% P/S + 0.1% BSA was added. At day 8, the medium was removed. 500µl of fresh DMEM 2mM L-Glutamine, 1g/L of glucose + 1% P/S + 0.1% BSA containing 2ng/ml of IL-1β ± Liraglutide pre-formulation or Victoza^{®} were added in each well according to Table 8. The plates were incubated at 37°C + 5% CO₂ for 24 hours.

**Table 8 - Study design**

| **Group** | **Treatment** | **Induction** | **Time** |
|---|---|---|---|
| 1 | Cells only (Blank) | Vehicle (Water) | 24 hours co-treatment |
| 2 | Vehicle (PBS) | | |
| 3 | Pre-formulation 2 4.1nM | | |
| 4 | Pre-formulation 2 12.3nM | | |
| 5 | Pre-formulation 2 37.0nM | | |
| 6 | Pre-formulation 2 111.1nM | | |
| 7 | Pre-formulation 2 333.3nM | | |
| 8 | Pre-formulation 2 1µM | | |
| 9 | Pre-formulation 2 3µM | | |
| 10 | Pre-formulation 6 4.1nM | | |
| 11 | Pre-formulation 6 12.3nM | | |
| 12 | Pre-formulation 6 37.0nM | IL-1β 2ng/ml | |
| 13 | Pre-formulation 6 111.1nM | | |
| 14 | Pre-formulation 6 333.3nM | | |
| 15 | Pre-formulation 6 1µM | | |
| 16 | Pre-formulation 6 3µM | | |
| 17 | Victoza^{®} 4.1nM (in PBS) | | |
| 18 | Victoza^{®} 12.3nM (in PBS) | | |
| 19 | Victoza^{®} 37.0nM (in PBS) | | |
| 20 | Victoza^{®} 111.1nM (in PBS) | | |
| 21 | Victoza^{®} 333.3nM (in PBS) | | |
| 22 | Victoza^{®} 1µM (in PBS) | | |
| 23 | Victoza^{®} 3µM (in PBS) | | |

Each condition treatment was run in quadruplicate.

**Table 9 - Study schedule**

| Procedure | Study day | | | | | | |
|---|---|---|---|---|---|---|---|
| | Before study | 1 | 4 | 7 | 8 | 9 | After study |
| Isolation of murine articular cartilage | √ | | | | | | |
| Isolation of immature murine chondrocytes | √ | | | | | | |
| Plating of immature murine chondrocytes | | √ | | | | | |
| Change medium | | | √ | | | | |
| Change medium containing BSA | | | | √ | | | |
| Stimulation with IL-1β +/- Pre-formulation Victoza^{®} or vehicle | | | | | √ | | |
| Collect the supernatant | | | | | | √ | |
| Dosages (NO) | | | | | | | √ |

### Tests and evaluations

At study termination, culture medium (± 500µl) of each well was collected in 1.5ml tube (1 tube per well), centrifuged at 4000rpm at room temperature and supernatant were added to a new 1.5ml tube. Samples were kept at 2-8°C for 2-3 days or frozen at - 70°C if Griess reagent Assay was not performed the same day.

As expected, NO production in vehicle conditions was below the limit of detection and were attributed a value of 1.56µM. In IL-1β-stimulated conditions, detection of nitrite oxide in culture medium is significantly increased compared to the vehicle. This secretion was significantly reduced by each tested-dose of Liraglutide pre-formulations or Victoza^{®} compared to the IL-1β alone condition, with a sigmoidal dose response pattern. IC₅₀ was calculated for each formulation using Prism software and summary results are presented in **Table 10.**

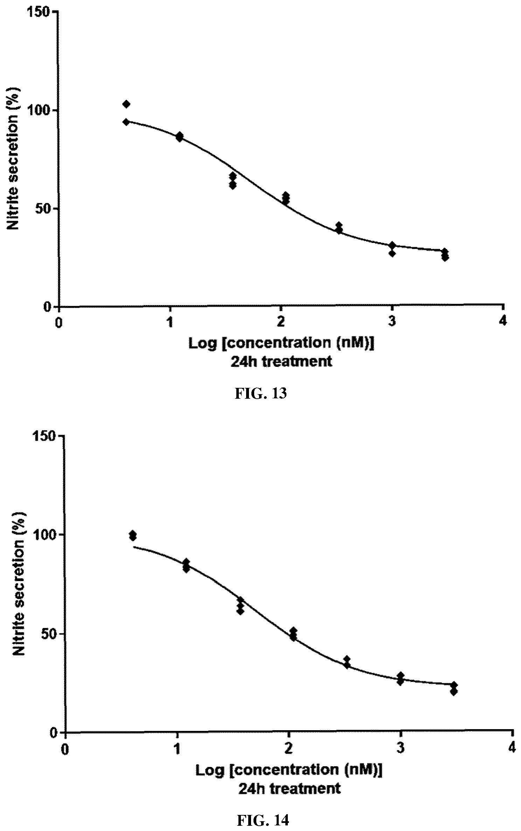

For murine primary chondrocytes cultured in low glucose conditions, Liraglutide pre-formulation 2 IC₅₀ dose on NO secretion is 60nM with a confidence interval between 53nM and 69nM (Figure 12); Liraglutide pre-formulation 6 IC₅₀ dose on NO secretion is 55nM with a confidence interval between 46nM and 64nM (Figure 13) and Victoza^{®} IC₅₀ dose on NO secretion is 52nM with a confidence interval between 46nM and 59nM (Figure 14).

**Table 10 - IC₅₀ summary table**

| **Formulation** | **IC50 value** |
|---|---|
| Victoza^{®} (PBS) | 52nM (46-59) |
| Pre-formulation 2 | 60nM (53-69) |
| Pre-formulation 6 | 55nM (46-64) |

### CONCLUSION

The objective of the study was to test 7 doses of 2 selected Liraglutide pre-formulations in comparison to Victoza^{®}, a marketed Liraglutide drug (4.1nM to 3µM) in murine primary chondrocytes. We demonstrated that Liraglutide pre-formulations 2 and 6 or Victoza^{®} test items were able to dose-dependently inhibit the IL-1β-induced NO production in murine primary chondrocytes. Liraglutide pre-formulations 2 and 6 and Victoza^{®} IC₅₀ dose on NO production in murine primary chondrocytes model in IL-1β-stimulated conditions was calculated. We confirmed that the two tested Liraglutide pre-formulations have overall the same anti-inflammatory and anti-degradative effect as Victoza^{®}.

The *in vitro* efficacy of compositions 2 and 6 is demonstrated, with an average IC50 value of 52nM to 60nM.

### Example 4: Efficacy of a single intra-articular (IA) knee injection of 3 ascending doses of preformulations-2 and -6 utilizing a MIA-induced model of osteoarthritis and inflammatory pain in rats

### Test system

Sprague Dawley Rat.

### Test items

**Table 11**

| **Pre-formulation name** | **Tonicity modifier** | **Buffer agent** | **Liraglutide API (6mg/ml)** |
|---|---|---|---|
| Pre-formulation 2 | Glucose | Tromethamine | yes |
| Pre-formulation 6 | PEG400 | Tromethamine | yes |

### Formulations:

Pre-formulations of Liraglutide (2 and 6) supplied as solutions at 6mg/ml were diluted in PBS to reach the final concentrations of 3,3µg, 20µg and 120µg in 30 µL.

Victoza^{®} is a commercial Liraglutide drug. Test item supplied as a solution at 6mg/ml was diluted in PBS to reach the final concentrations of 20µg in 30 µL.

The vehicle (PBS) is supplied "ready to use" and were diluted in cell seeding medium at a final concentration of 1:100.

### EXPERIMENTAL DESIGN

### OA induction by intra-articular (IA) injection of MIA (monosodium iodoacetate)

Animals were anesthetized via a chamber induction technique using inhalation anesthesia (Isoflurane at 5%). During the procedure, the animal was maintained under Isoflurane at a level between 1.5 and 3% with an air flow rate of 1-2 liters/minute. After induction of anesthesia, the right hind limb skin surface was clipped free of hair using electric animal clippers. After shaving, the area surrounding the knee joint was wiped with alcohol. A volume of 30µL containing 3mg MIA (monosodium iodoacetate) was injected intra-articularly (IA) in the knee joint through the patellar tendon. A 29-Gauge, 0.5-inch needle that was fitted with cannulation tubing was used such that only 3 to 4 mm of the needle was allowed to puncture the joint. After injection, the knee was massaged to ensure even distribution of the solution. Animals were injected once on day 1 (groups 2M, 3M, 4M, 5M, 6M, 7M, 8M, 9M). For group 1M (sham control), 30µL of injectable saline was injected into knee joint.

### Mechanical pain evaluation (Von Frey test)

Von Frey test in the rats following MIA (monosodium iodoacetate) injection was conducted according to 4P-Pharma's Standard Operating Procedures (SOP). Rats were placed individually in the designated transparent Plexiglas chambers and allowed to acclimate for 10-15 min. Withdrawal responses to mechanical stimulation were determined using electronic von Frey apparatus (BIO-EVF5, Bioseb) with tip of 0,5 mm diameter on the stimulator handle applied from underneath the cage through openings (12x12 mm) in the plastic mesh floor. The withdrawal response in gram-force for each rat was calculated from three trials. Von Frey Test was performed on the animals at day 2 (as baseline before treatment, for group allocation), day 7 and day 10 (4 and 7 days following treatment, respectively): total of three times. The experimenter(s) was/were blind regarding the identity of the groups.

**Table 12 - Group allocation**

| **Group** | **N=** | **MIA induction (3mg in 30µL)** | **Treatment** | **Dose level** | **Dose volume** | **ROA** | **Dose regimen** |
|---|---|---|---|---|---|---|---|
| 1M | 8 | - | Vehicle (PBS) | - | 30µL | IA | Once on day 3 |
| 2M | 8 | √ | Vehicle (PBS) | - | | | |
| 3M | 8 | √ | Victoza^{®} | 20µg | | | |
| 4M | 8 | √ | Pre-formulation 2 | 3,3µg | | | |
| 5M | 8 | √ | | 20µg | | | |
| 6M | 8 | √ | | 120µg | | | |
| 7M | 8 | √ | Pre-formulation 6 | 3,3µg | | | |
| 8M | 8 | √ | | 20µg | | | |
| 9M | 8 | √ | | 120µg | | | |

**Table 13 - Study timeline**

| **Study Day/week*** | **Procedure** | **Remarks** |
|---|---|---|
| **D1** | MIA injection, 3mg in 30µL, IA | Except for group 1M (sham control), 30µL injectable saline, IA |
| **D3** | Treatment (Vehicle, Victoza^{®} or Liraglutide pre-formulations) | IA single injection, 30µL |
| **D2, D7, D10** | Von Frey test | |
| **D11** | Termination | Right knee (diseased) collection |

### Statistical plan

For statistics, we applied a sequential testing strategy reflecting experimental expectations, while retaining the central Mann-Whitney (non-parametric) test. Sequential statistics were made to compare in this order:
1. The control/vehicle group (1M) and the MIA/vehicle groups (2M).
2. The MIA/vehicle groups (2M) and the MIA/Victoza^{®}-treated animals (20µg) (3M).
3. The MIA/vehicle groups (2M) and the higher dose of preformulation-2 or -6 (120µg) (6M and 9M). Subsequently, we compare the MIA/vehicle group (2M)

### TESTS AND EVALUATIONS

### Mechanical pain evaluation (Von Frey test)

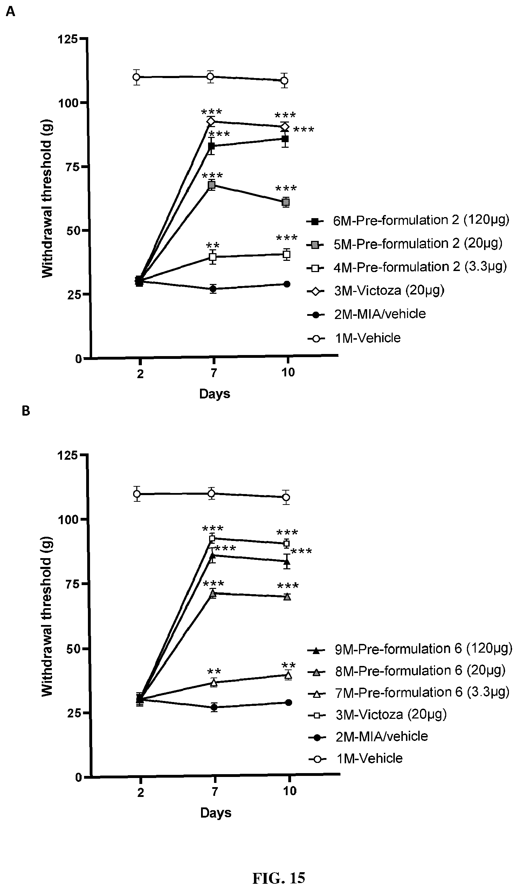

Allodynia in rats following OA induction on day 1 was evaluated on day 2 as baseline for group allocation and then on days 7 and 10 to assess effect of treatments on mechanically-induced pain. For each group, mean of withdrawal responses, as given by the electronic von Frey apparatus, for the left and right hind paws, was calculated. Group average animal withdrawal response results are presented in **Table 14** for the right hind paw. Results on the right hind paw are presented in Figure 15. Figure 15A compares vehicle control, MIA/vehicle and MIA/Victoza^{®}-groups (1M-3M) to the three groups of Preformulation-2 (4M-6M), Figure 15B compares vehicle control, MIA/vehicle and MIA/Victoza^{®}-groups (1M-3M) to the three groups of Preformulation-6 (7M-9M).

On day 7, there was a significant increase of paw withdrawal threshold in MIA-injected animals between Victoza^{®}-treated group 3M vs MIA/vehicle group 2M (mean ± SD; p<0.001). A significant dose effect was observed in preformulations groups at 120 µg (9M and 6M), 20 µg (8M and 5M), and 3,3 µg (7M and 4M) vs MIA/vehicle group 2M (mean ± SD; p<0.001 for 120 and 20 µg doses and mean ± SD; p<0.01 for 3,3 µg dose) on day 7.

A significant decrease on the withdrawal threshold from D7 to D10 was observed using the 20 µg of preformulation-2 (5M) (mean ± SD; p<0.05). The withdrawal threshold effect lasted from D7 to D10 using the preformulation-2 at 3,3 and 120 µg (4M and 6M) and the preformulation-6 (7M, 8M and 9M) since non-statistical difference was observed from D7 to D10 on these groups (4M, 6M, 7M, 8M and 9M). Higher paw withdrawal threshold was observed when comparing 20 µg of Victoza^{®} IA injection and of 20 µg of preformulation-2 and -6.

**Table 14 - Summarized averages of right paw withdrawal threshold in grams (mean ± SD)**

| **Group** | **Treatment** | **D2** | | **D7** | | **D10** | |
|---|---|---|---|---|---|---|---|
| | | **Mean** | **SD** | **Mean** | **SD** | **Mean** | **SD** |
| **1M** | **Vehicle** | 109,62 | 8,37 | 109,43 | 6,54 | 107,62 | 7,96 |
| **2M** | **MIA/Vehicle** | 29,85 | 5,63 | 26,45 | 4,74 | 28,00 | 3,13 |
| **3M** | **MIA/Victoza (20µg)** | 29,92 | 3,65 | 91,93*** | 5,62 | 89,56*** | 5,13 |
| **4M** | **MIA/Preformulation-2 (3.3µg)** | 30,09 | 5,09 | 38,98** | 8,01 | 39,71*** | 6,38 |
| **5M** | **MIA/Preformulation-2 (20µg)** | 29,93 | 4,51 | 67,20*** | 6,07 | 60,05*** | 5,43 |
| **6M** | **MIA/Preformulation-2 (120µg)** | 30,07 | 5,15 | 82,24*** | 9,52 | 84,88*** | 9,42 |
| **7M** | **MIA/Preformulation-6 (3.3µg)** | 29,99 | 4,32 | 36,10** | 4,99 | 38,78** | 5,60 |
| **8M** | **MIA/Preformulation-6 (20µg)** | 30,10 | 7,19 | 70,72*** | 5,28 | 69,13*** | 2,93 |
| **9M** | **MIA/Preformulation-6 (120µg)** | 29,75 | 5,32 | 85,31*** | 8,36 | 82,64*** | 7,99 |

| | | | | | | | |
|---|---|---|---|---|---|---|---|
| MIA/Vehicle (2M) vs MIA preformulation-2 and -6 (4M, 5M, 6M, 7M, 8M and 9M) (Mann-Whitney test; ** p<0.01, *** p <0,001). | | | | | | | |

### CONCLUSION

In conclusion, in this comparative study between IA injection of 20µg of Victoza^{®} and three doses (3.3, 20 and 120 µg) of preformulation-2 and -6 we observed that both preformulations display dose-dependent analgesic effects.

### Example 5: Efficacy of intra-articular (IA) knee injection of Victoza^{®} utilizing a MIA-induced model of osteoarthritis and inflammatory pain in rats

### Objective:

The objective of the present study was double: to perform a dose response study using Victoza^{®} utilizing a MonoIodoAcetate (MIA)-induced model of osteoarthritis and inflammatory pain in rats to calculate a half maximal effective concentration EC50 (short term MIA model) and to determine on 5 selected groups the duration of the analgesic effect after a single intra-articular administration of Victoza^{®} (long term MIA model). Dexamethasone was used as a reference positive-control group in the study.

### Method:

Nine groups comprising 8 SD rats per group were allocated for this study performed in 4 cycles. Chemically-induced OA disease was performed on eight out of nine groups. On day 1, 3mg of MIA in 30µL was injected intra-articularly (IA) in the right knee joint. The last group (sham control) received 30µL of saline IA in the right knee joint. The nine groups were as follows: one sham control group (1M) treated with vehicle (water) and eight MIA groups treated with vehicle (2M), Victoza^{®} at 07µg (3M), 2.2µg (4M), 6.7µg (5M), 20µg (6M), 60µg (7M) or 180µg (8M) and dexamethasone at 120µg (9M). All animals were injected IA once on day 3. During the study, mortality and morbidity observation and von Frey tests using electronic von Frey apparatus were performed. Group allocation was performed on day 2, based on von Frey results. Knee harvesting was performed for optional analyses at study termination (day 11 for short term MIA model or day 32 for long term MIA model).

### Results:

### EC50, short term MIA model (up to day 11) for all groups:

Mortality/morbidity: No mortality nor morbidity was observed during the study.

Von Frey test: As expected, there was a significant decrease of paw withdrawal threshold following MIA injection as compared to sham control (group 1M), confirming model induction. This effect lasted until study termination for group 2M. On day 7, a significant dose-dependent increase in paw withdrawal threshold was observed between MIA/Victoza^{®}-treated groups 5M, 6M, 7M and 8M as well as group 9M treated with dexamethasone 120µg vs MIA/vehicle group 2M. Calculated EC50 was 3.3µg. On day 10, a significant dose-dependent increase in paw withdrawal threshold was observed between MIA/Victoza^{®}-treated groups 4M, 5M, 6M, 7M and 8M as well as group 9M (dexamethasone) vs MIA/vehicle group 2M. Calculated EC50 was 2.1µg.

### Long term MIA model (up to day 32) for 5 selected groups:

The 5 selected groups were: 1M (sham/vehicle), 2M (MIA/vehicle), 7M (MIA/Victoza^{®} 60µg), 8M (MIA/Victoza^{®} 180µg) and 9M (MIA/dexamethasone).

Mortality/morbidity: No mortality or morbidity was observed during the study.

Von Frey test: After day 10 (i.e. one week after acute treatment), von Frey tests were performed once a week at day 18, 25, 31 until study termination. The significant decrease of paw withdrawal threshold following MIA injection lasted until study termination for group 2M as compared to sham control group 1M, confirming model induction and maintenance up to day 31. For the other groups, results indicated that the analgesic effect of Victoza^{®} 60µg (group 7M) and 180µg (group 8M) as well as the one of dexamethasone 120µg (group 9M) was maintained significantly up to day 25 (i.e. 3 weeks following acute administration) but was lost at day 31 (i.e. 4 weeks following acute injection). Results are presented in Figure 16.

### Conclusion:

In conclusion, this study confirms that locally-administered Victoza^{®} targets relevant mechanisms associated with pain in a MIA-induced OA and inflammatory pain model in rats. The calculated half maximal effective concentration is 2.1µg at day 7 and 3.3µg at day 10. Moreover, here, we demonstrate a 3 weeks long-lasting analgesic effect of Victoza^{®} following acute IA administration comparable to that of dexamethasone positive control.

### Example 6: Clinical trial phase 1

A Phase I Clinical Trial to Evaluate the Safety, Tolerability, Pharmacokinetics and Efficacy of single ascending doses of 4P004 versus placebo injected in the Target Knee Joint of Patients with Osteoarthritis within 3 to 6 months before total knee replacement. 4P004 refers to a composition according to the present invention, that is a composition that comprises GLP-1R agonist for IA injection.

### Objectives

### Primary objective

To assess the clinical and biological safety, and general and local tolerability of liraglutide when administered as knee intra-articular (IA), single ascending dose, in patients with severe knee osteoarthritis and scheduled for total knee replacement (TKR).

### Secondary objectives

To determine the plasma PK of liraglutide when administered as single IA doses at escalating dose levels in patients scheduled for TKR.

### Exploratory Objectives

To evaluate histological and molecular data on the different parts of the knee after removal.

### Endpoints

### Primary endpoints

1. Difference between 4P004 treated subjects and placebo in the number of Adverse Events [Time Frame: from screening until final follow-up visit]
2. Difference between 4P004 treated subjects and placebo in the number of Abnormal vital signs [Time Frame: from screening until final follow-up visit]
3. Difference between 4P004 treated subjects and placebo in the number of Abnormal clinical laboratory evaluations [Time Frame: from screening until final follow-up visit]
4. Difference between 4P004 treated subjects and placebo in the number of abnormal physical examination [Time Frame: from screening until final follow-up visit]

### Secondary endpoints

Evaluation of PK parameters: (Cmax, Tmax, AUC0-t, AUCO-∞, T1/2) Day 1 and Day 2.

### Overall design

This phase I is a randomized, double-blind, placebo-controlled study to assess the safety and tolerability of single ascending dose of intra-articular 4P004 at 0.2 mg, or 0.6 mg, 1.8mg and 6 mg in patients
- between 18 and 80 years of age,
- with severe knee osteoarthritis,
- scheduled for TKR within 3 (>3) to 6 months.

A total of 32 participants will be enrolled in 4 cohorts, each cohort will receive either 4P004 or placebo (6:2). 4P004 dose will increase with cohort 1 to 4.

### Inclusion criteria

- Patients who have the capacity to give informed consent and who are willing to comply with all study related procedures and assessments (consent via legally authorized representative will not be accepted)
- Patients must be ≥ 18 and ≤ 80 years of age
- Patients diagnosed with primary osteoarthritis of the knee assessed locally for whom a total knee replacement is scheduled within 3 to 6 months.

### Exclusion criteria

- Treatment with systemic glucocorticoids greater than 10 mg prednisone or the equivalent per day within 4 weeks prior to screening
- Any known active infections
- Any chronic condition that has not been well controlled for a minimum of 3 months
- History of malignancy of any organ system (other than localized basal cell carcinoma of the skin or in-situ cervical cancer) within the last 2 years
- Diabetes type I and type II patients managed by GLP-1 analogues
- Patients exposed to Glucagon-peptide 1 analog hormones
- Treatment of the target knee with intra-articular injection (steroids, hyaluronic acid derivatives ....) within 3 months
- Use of topical analgesic agents (gels, creams, or patches) for the treatment of knee OA within 7 days of screening
- Effusion of the target knee requiring aspiration within 3 months
- Use of electrotherapy or acupuncture for OA within 4 weeks
- Significant and clinically evident misalignment of the target knee
- Any condition, including laboratory findings, that in the opinion of the investigator constitutes a risk or contraindication for participation in the study or that could interfere with the study objectives, conduct or evaluation
- Participation in a clinical research trial within 12 weeks prior
- Hypersensitivity to the active substance or to any of the excipients: Disodium phosphate dihydrate, Propylene glycol, Phenol.

### Results

We expect to demonstrate that 4P004 is safe and well tolerated in human for a dose within the tested doses. And we expect to determine the pharmacokinetics parameters of a single intra-articular injection the patients knee joint.

### Example 7: Clinical trial phase 2

### PHASE II STUDY

Dose ranging study to assess the efficacy and safety of 4P004 administered via intraarticular injection in patients with mild to moderate osteoarthrosis of the knee. 4P004 refers to a composition according to the present invention, that is a composition that comprises GLP-1R agonist for IA injection.

### Objectives

### Primary objective

To demonstrate the efficacy of 4P004 in knee osteoarthritis and to determine the optimal dose regimen

### Secondary objectives

To assess the safety and tolerability of 4P004 different dose regimen versus placebo

To assess complementary clinical efficacy of 4P004 versus placebo

### Exploratory objectives

Biomarkers
Clinical signs of structural change by imaging

### Endpoints

### Primary endpoint

To demonstrate significant pain improvement using Western Ontario and McMaster Universities Osteoarthritis Index (WOMAC) pain score of the knee versus placebo at 3 months

### Change from baseline in OMERACT-OARSIS score

### Secondary endpoints

- Clinical and laboratory safety
- Change from baseline pain using a Visual Analog Scale (VAS) For phase II study participants
- Change from baseline per physician global assessment of disease scale
- Change from baseline patient global assessment (PGA)
- Change from baseline in OMERACT-OARSIS score
- Change from baseline in MRI-assessed synovitis at 6 and 12 months
- Change in joint space narrowing in the target knee.

### Inclusion Criteria

- Male or female between 18 and 80 years of age,
- Using contraceptive consistent with local regulations regarding the methods of contraception for those participating in clinical studies,
- Willing and able to provide written informed consent
- Established Clinical diagnosis of OA in the target knee for at least 6 months (clinical and x-ray criteria)
- Radiographic (x-ray) disease Stage 2 or 3 in the target knee according to the Kellgren-Lawrence grading of the knee OA
- Primary source of pain throughout the body is due to OA in the target knee
- pain visual analog scale (VAS) score of 30-80 mm (on 100-mm VAS) and a WOMAC total
- score of 72-192 (of 240) for the target knee at screening
- ambulatory; assistive devices (e.g., canes) were allowed if needed <50% of the time, whereas any use of a walker was excluded
- Body mass index < 40

### Exclusion Criteria

- Major knee surgery in the target knee within 12 months prior to study or planned surgery during the study period
- Partial or complete joint replacement in the target knee
- Currently requires regular use of ambulatory assistive devices (e.g wheelchair, parallel bars, walker, canes or crutches)
- Comorbid conditions that could affect study endpoint assessments of the target knee, including, but not limited to, Rheumatoid arthritis, psoriatic arthritis, systemic lupus erythematosus, gout or pseudogout, and fibromyalgia.
- Diabetes type I and type II patients
- Patients exposed to Glucagon-peptide 1 analog hormones
- History of malignancy within the last 3 years
- Participation in a clinical research trial within 12 weeks prior
- Treatment of the target knee with intra-articular steroids within 2 months or hyaluronic acid derivatives within 6 months
- Treatment with systemic glucorticoids greater than 10 mg prednisone or the equivalent per day within 4 weeks prior to screening
- Effusion of the target knee requiring aspiration within 3 months
- Use of electrotherapy or acupuncture for OA within 4 weeks
- Significant and clinically evident misalignment of the target knee
- Any known active infections
- Any chronic condition that has not been well controlled for a minimum of 3 months
- Use of centrally acting analgesics (e.g duloxetine) within 12 weeks prior to screening
- Use of anticonvulsants within 12 weeks prior to screening, unless used for seizure or migraine prophylaxis
- Use of topical analgesic agents (gels, creams, or patches) for the treatment of knee OA within 7 days of screening
- Any condition, including laboratory findings, that in the opinion of the investigator constitutes a risk or contraindication for participation in the study or that could interfere with the study objectives, conduct or evaluation

### Overall design

This is a dose range finding, randomized, double-blind and placebo-controlled study to assess the efficacy and safety 3 dose of intra-articular 4P 004 at 3 ascending doses or placebo in patients
- between 18 and 80 years of age,
- with mild or moderate knee osteoarthritis,

Approximately 500 participants will be randomized in one of the 4 treatment groups to receive one of the 3 fixed doses of 4P004 or placebo.

**Results**

## Claims

1. A pharmaceutical composition for use in a method for treating joint diseases, in particular osteoarthritis and/or joint pain, more particularly inflammatory joint pain,
wherein said pharmaceutical composition is to be administered via intraarticular injection, in particular via intraarticular injection into the joint cavity, and wherein said pharmaceutical composition comprises:
- a GLP-1R agonist,
- a buffer selected from the group consisting of a tromethamine buffer and a phosphate buffer, and
- an isotonic agent selected from the group consisting of glucose, a polyethylene glycol, propylene glycol and glycerol.

2. The pharmaceutical composition for use according to claim **1,** wherein the GLP-1R agonist is liraglutide, the buffer is a phosphate buffer, preferably a phosphate buffer comprising disodium phosphate dihydrate as buffering agent, and the isotonic agent is propylene glycol.

3. The pharmaceutical composition for use according to claim **1,** wherein said pharmaceutical composition comprises:
- a GLP-1R agonist, preferably the GLP-1R agonist is liraglutide,
- a buffer selected from the group consisting of a tromethamine buffer and a phosphate buffer, and
- an isotonic agent selected from the group consisting of glucose, a polyethylene glycol and glycerol.

4. The pharmaceutical composition for use according to any one of claims **1** to **3,** wherein the GLP-1R agonist is liraglutide and wherein said pharmaceutical composition is to be administered at a dose from 0.0245 mg to 6.3 mg of liraglutide, preferably at a dose from 0.7 mg to 6.3 mg, of liraglutide.

5. The pharmaceutical composition for use according to any one of claims **1** to **4,** wherein said dose of said pharmaceutical composition is to be administered in one or at least two intraarticular injections.

6. The pharmaceutical composition for use according to any one of claims **1** to **5,** wherein doses of said pharmaceutical composition are to be administered every month.

7. The pharmaceutical composition for use according to any one of claims **1** to **6,** wherein the total dose of GLP-1R agonist that is administered in one year is from 0.18 mg to 72 mg, preferably from 0.7 mg to 8.4 mg.

8. A pharmaceutical composition comprising:
- a GLP-1R agonist,
- a buffer selected from the group consisting of a tromethamine buffer and a phosphate buffer, and
- an isotonic agent selected from the group consisting of glucose, a polyethylene glycol and glycerol.

9. The pharmaceutical composition according to claim **8,** wherein the GLP-1R agonist is liraglutide.

10. The pharmaceutical composition according to claim **8** or **9,** wherein the pharmaceutical composition comprises from 2 to 20 mg/mL, preferably from 4 to 8 mg/mL, more preferably about 6 mg/mL of GLP-1R agonist.

11. The pharmaceutical composition according to any one of claims **8** to **10,** wherein the buffer is a tromethamine buffer comprising tromethamine as buffering agent, preferably the pharmaceutical composition comprises from 0.1 to 10 mg/mL, more preferably from 0.5 to 1 mg/mL, even more preferably about 0.97 mg/mL of tromethamine.

12. The pharmaceutical composition according to any one of claims **8** to **10,** wherein the buffer is a phosphate buffer comprising disodium phosphate as buffering agent, preferably the pharmaceutical composition comprises from 0.1 to 10 mg/mL, more preferably from 0.75 to 1,5 mg/mL, even more preferably about 1,14 mg/mL of disodium phosphate.

13. The pharmaceutical composition according to any one of claims **8** to **12,** wherein the isotonic agent is glucose, preferably said pharmaceutical composition comprises from 10 to 50 mg/mL, more preferably from 20 to 40 mg/mL, even more preferably about 30 mg/mL of glucose.

14. The pharmaceutical composition according to any one of claims **8** to **13,** wherein the isotonic agent is a polyethylene glycol having a molecular weight less than 800 g.mol⁻¹, preferably from 100 g.mol⁻¹ to 600 g.mol⁻¹, preferably the isotonic agent is PEG400, and
wherein said pharmaceutical composition comprises from 20 to 100 mg/mL, preferably from 40 to 80 mg/mL, more preferably about 60 mg/mL of polyethylene glycol.

15. The pharmaceutical composition according to any one of claims **8** to **14,** wherein said pharmaceutical composition comprises from 5 to 50 mg/mL, preferably from 10 to 25 mg/mL, more preferably about 17 mg/mL or about 18 mg/mL of glycerol.
